# EUROPEAN PATENT APPLICATION

(11) **EP 1 884 523 A1**
(43) Date of publication of application: **06.02.2008**
(21) Application number: 06118165.7
(22) Date of filing: 31.07.2006
(51) Int. Cl.: C07K 16/18, G01N 33/53, A61P 35/00, A61K 31/505

(54) **Biomarkers for polo-like kinase 1 activity**

(71) Applicant: Boehringer Ingelheim International GmbH, 55216 Ingelheim am Rhein (DE)
(72) Inventor: Hegemann, Bjoern, 1020 Wien (AT); Peters, Jan-Michael, 2103 Langenzersdorf (AT); Hudecz, Otto, 3400 Klosterneuburg (AT); Hutchins, James R. A., 1050 Wien (AT); Kueng, Stephanie, 8173 Neerach (CH); Mechtler, Karl, 2126 Ladendorf (AT)
(74) Representative: Hammann, Heinz

(57) **Abstract**

Antibodies recognizing Plk1-dependent phosphorylation of the cohesion subunits Scc1 or SA2 and methods for using such antibodies for determining Plk1 activity, e.g. in clinical trials for assessing the therapeutic effect of Plk1 inhibitors.

## Description

The present invention relates to the therapy of cancer with inhibitors of Polo-like kinase 1 (P1k1), in particular to methods for determining the activity of P1k1 in human cells.

The cell cycle has been recognized as a major target of therapeutic intervention in the treatment of cancer. Therefore, inhibitors that interfere with proteins of the cell cycle have gained increasing importance as anti-cancer drugs.

Anti-cancer drugs are sometimes judged for their effect on a surrogate marker (also termed "biomarker") that may predict clinical benefit to the patient, such as regression of tumors. When monitoring a patient's response to a cell cycle inhibitor, a surrogate marker is the basis for clear demonstration that an administered compound and a chosen dose and treatment schedule have reached levels sufficient to inhibit the activity of the cell cycle target.

However, traditional clinical end points have proven difficult to apply in the evaluation of molecularly targeted therapies; standard clinical trial endpoints that are used for cytotoxic compounds have been found to be insufficient for the evaluation of molecularly targeted, e.g. cell cycle-targeted, antiproliferative agents.

In the development of molecularly targeted, e.g. cell cycle-targeted, inhibitors, it is important to have endpoint assessments available for (i) the efficacy of the compound in modulating the activity of its molecular target, and (ii) the relationship between target modulation and clinical response.

Thus, the availability of biomarkers (i.e. a measurable biological activity that indirectly indicates the effect of treatment on disease state) is one of the key prerequisites for the development of minimally invasive techniques to assess an inhibitor's efficacy and, consequently, both for the clinical evaluation of the drug and, in the long run, for monitoring the efficacy of the approved drug in therapy.

The molecularly targeted agent STI-571 (the Novartis compound also known as Gleevec) is a positive example for the contribution of a surrogate marker to successful clinical development: STI-571 was developed as a tyrosine kinase inhibitor with specificity for the Abl tyrosine kinase, although c-Kit and the platelet-derived growth factor receptor are also targets. In chronic myelogenous leukemia, 95% of patients have the Philadelphia chromosome that results in generation of the oncogenic Bcr-Abl fusion protein. Studies have shown that the activity of this oncogenic kinase is sufficient to cause the disease, thus allowing therapeutic efforts to be focused on a highly appropriate patient population (Druker and Lydon, 2000). Inhibition of tyrosine kinase activity was readily assayed by the analysis of levels of phospho-CrkL, the predominant Bcr-Abl substrate in neutrophils. This endpoint assay assisted in the identification of a biologically active dose. The rapid and successful clinical development of Gleevec largely rests on the fact that the disease in the patient population tested was dependent on ABL activity, and there were methods available for the determination of ABL inhibition and disease remission in patients.

Another example of a cell cycle target whose activity can be readily measured is CDK4; this assay employs detection of pRB phosphorylation by CDK4 (Fry, et al., 2001). Western blot analysis of lysates prepared from compound-treated cells were performed using an antibody that detected phosphorylation of pRb at serine 780, a site that is thought to be phosphorylated by CDK4.

There are many examples of substrate phosphorylation sites that can serve as useful readouts for cellular inhibition of a particular molecular target. With reference to the cell cycle in particular, phosphorylation on serine 216 of the dual specificity phosphatase Cdc25C has been used to monitor the cellular activity of the Chk1 cell cycle checkpoint kinase (Graves, et al., 2000). Similarly, phosphorylation of the core histone H3 on serine 10 can be used to monitor the efficacy of inhibitors of the kinase Aurora B (Ditchfield et al., 2003; Hauf et al., 2003).

Furthermore, Wee 1 activity has been examined by detection of phosphorylation on tyrosine 15 and threonine 14 on CDK1 using phosphospecific antibodies to these sites (Wang, et al., 2001).

Importantly, total substrate levels as well as the level of phosphorylation on a particular site should always be determined to monitor specific signal loss (Fry, et al., 2001).

Plk 1 is another cell cycle kinase that has been considered as a target for anti-cancer therapy. This is due to increasing evidence that P1k1 forms part of the regulatory circuit that controls entry into and progression through mitosis. P1k1 belongs to the family of serine/threonine kinases, and it has been shown to be required for spindle formation and chromosome segregation during mitosis. The essential role of P1k1 orthologues in controlling the progression of dividing cells through mitosis has been demonstrated by genetic means in several model organisms (e.g., Drosophila melanogaster, Xenopus laevis, yeast) as well as with antibody injection or siRNA knock-down experiments in human tumor cells (Llamazares et al., 1991; Kitada et al., 1993; Lane and Nigg, 1996; Spänkuch-Schmitt, et al., 2002; Liu and Erikson, 2002; Liu and Erikson, 2003; Sumara et al., 2004; van Vugt et al., 2004). It has also been shown that P1k1 mRNA expression correlates with the mitotic activity of cells and the prognosis of lung cancer patients (Yuan, et al., 1997). Inhibitors of P1k1 are therefore thought to be promising drugs for the treatment of neoplastic diseases (Strebhardt and Ullrich, 2006). However, to date no specific biomarkers and, consequently, no assays are available to directly monitor the effect of such a drug on the cellular activity of P1k1. Thus, in order to determine the in vivo efficacy of P1k1 inhibitors and to select patients for being treated with P1k1 inhibitors, there is a need for P1k1 biomarkers.

It was therefore an object of the invention to provide reliable biomarkers for assessing P1k1 activity and, in particular, for selecting patients for treatment with a P1k1 inhibitor and for assessing a drug's effect on the activity of P1k1 during therapy.

The solution of the problem underlying the invention is based on the consideration that a desirable biomarker would be one or more protein phosphorylation sites whose generation in vivo depends on P1k1 activity. If such sites can be identified, antibodies can be raised against them and can be used in different immunological assays to determine the presence or absence of P1k1 activity in cells and tissues.

At present, only very few phosphorylation sites are known whose generation is thought to depend on P1k1 (reviewed in Barr et al., 2004), but none of these have been rigorously validated as sites specifically modified by P1k1 in vivo.

In mitosis, the cohesin complex is phosphorylated on its subunits Scc1 and SA2, and the amino acid residues that are phosphorylated in these proteins have been identified by mass spectrometry (Hauf et al., 2005). In whole cell extracts of Xenopus eggs, which recapitulate cell cycle reactions in a physiological manner, these phosphorylation reactions depend on the Xenopus ortholog of P1k1, called P1x1 (Sumara et al., 2002). Both in Xenopus egg extracts and in living human cells the dissociation of cohesin from chromosome arms during prophase and prometaphase of mitosis depends on P1x1 and P1k1, respectively (Sumara et al., 2002; Losada et al., 2002; Giménez-Abian et al., 2004). The dissociation of cohesin from chromosome arms also depends on the phosphorylation of SA2 (Hauf et al., 2005). Finally, P1x1/P1k1 is able to phosphorylate cohesin in vitro (Losada et al., 2002; Sumara et al., 2002). Based on these observations it has been speculated that cohesin is phosphorylated by P1k1 in human cells in vivo (Hauf et al., 2005). However, direct evidence for this hypothesis has so far been lacking. In particular, it was unknown which of the previously identified phosphorylation sites on Scc1 and SA2 are dependent on P1k1 activity.

To solve the problem underlying the invention, the inventors sought to identify the phosphorylation sites that are specific for P1k1 activity. In a first step, the inventors re-addressed the question of cohesin phosphorylation:
For Scc1 (SEQ ID NO: 1), Hauf et al. 2005 describe phosphorylation sites at serine 46, serine 138, threonine 144, serine 153, serine 175, serine 185 or threonine 186 or threonine 187 or threonine 188 or serine 189, threonine 312, serine 454, serine 459 and serine 466. In the experiments of the invention, additional phosphorylation sites at serine 271 or serine 277, at serine 280, at serine 545 and at threonine 623 of SEQ ID NO:1 were detected. Serine 138 could not be unambiguously identified as phospho site; it may also be the case that the nearby serine 134 is the relevant site to be phosphorylated. The phosphorylation sites at serine 185 or threonine 186 or threonine 187 or threonine 188 or serine 189, serine 454, serine 459 and serine 466 found by Hauf et al. 2005 could not be detected. This difference may be due to the fact that the purification and the mass spectrometry experiments of the invention were carried out with a protocol and on a mass spectrometer (the LTQ from Thermo Finnigan) that were different than the protocol and instrument (the LCQ from Thermo Finnigan) used in the study by Hauf et al., 2005.
For SA2 (SEQ ID NO:2) Hauf et al. 2005 identified phosphorylation sites at serine 377 or threonine 379, serine 843, serine 1058, serine 1064 or serine 1065, threonine 1109 or threonine 1112, threonine 1118, serine 1123 or threonine 1124, serine 1137 or serine 1140, threonine 1146, threonine 1151, threonine 1160 or serine 1161, serine 1177 or serine 1178, threonine 1193 and serine 1224.
   In the experiment of the invention phosphorylation sites at serine 377 or 379, serine 1058, serine 1064 or serine 1065, threonine 1118, serine 1123 or threonine1124, serine 1137 or serine 1140, threonine 1146 and threonine 1193 could not be detected. As stated above for Scc1, this difference may be due to the different experimental protocols used. Threonine 1160 and serine 1224 could not be unambiguously identified as phospho sites; it may also be the case that the nearby serine 1160 or serine 1229, respectively, is the relevant site to be phosphorylated.

Next, the inventors sought to determine which of the phosphorylation sites identified on Scc1 and SA2 depend on P1k1 activity in vivo and could thus be useful as biomarkers for P1k1 activity. To address these questions, the inventors developed a method that combines chemical biology, protein affinity purification and mass spectrometry. This method, which was used in the experiments of the invention to determine the P1k1-dependent phosphorylation sites on Scc1 and SA2, can be used as a generic method to screen for biomarkers of various enzyme activities:
Since protein function is often regulated by post-translational modifications, e.g. phosphorylation, post-translational modifications on a protein can be used as readout for enzyme activity, i.e. as a biomarker. To be able to easily detect the phosphorylation state or another post-translational modification state of a target protein and to identify on which enzyme such modifications depend, the inventors developed the following method.

In a first step of this method, cultured cells, e.g. from a tumor cell line in which the enzyme of interest is active (in the case of the present invention the kinase P1k1), are treated with an inhibitor of the enzyme of interest, or left untreated (control cells). After a period of time that is sufficient for the inhibitor to exert its effect on the activity of the enzyme of interest, the target protein is isolated from both the inhibitor-treated and untreated cells by affinity purification and analysed by mass spectrometry under conditions suitable for identifying the post-translational modifications on the target protein. Post-translational modifications that are present on the untreated cells and that are absent on the inhibitor-treated cells are those that are dependent on the enzyme of interest and can thus serve as biomarkers.

Antibodies can then be generated against the identified modifications and can be used to monitor the enzyme's activity in cells and tissues.

The described method has wide applications in all relevant biological pathways in which enzymes post-translationally modify target proteins, provided such modifications can be detected by mass spectrometry. Examples for such modifications are: acetylation, alkylation, biotinylation, glycosylation, lipoylation, phosphorylation, sulfation, ubiquitination, sumoylation or proteolytic processing. In the case of the present invention, the novel method was used to detect phosphorylation sites on cohesin subunits that are dependent on the mitotic kinase

P1k1 by using the P1k1 inhibitor BI 2536 and a purification and mass spectrometry protocol designed to detect phosphorylation sites on the target proteins of P1k1. To detect phosphorylation sites generated by other kinases of interest, a range of inhibitors can be used (e.g. Hesperadin to inhibit Aurora B kinase (Hauf et al. 2003), Roscovitine to inhibit Cyclin dependent kinase 1 (Meijer L et al. 1997) or Gleevec to inhibit Abl tyrosine kinase.

The same principle can be applied to other post-translational modifications like acetylation using acetylase inhibitors (e.g. trichostatin A (Minucci S et al. 1997)) or methylation using methylase inhibitors (e.g. chaetocin (Greiner D et al. 2005). Both modifications are especially abundant on histone proteins and can be identified by mass spectrometry methods (Rice JC et al. 2003; Beck HC et al. 2006). Many other post-translational modifications can be detected by various mass spectrometry methods (reviewed in Cantin GT and Yates JR 3rd (2004)). In combination with the vast range of enzyme inhibitors, the method of the invention offers plenty possibilities to detect post-translational modification-dependent biomarkers. The protocol can be easily applied to any given target protein of interest by adapting standard affinity purification protocols to that protein, e.g. by providing the specific antibodies, and combining the purification method with the mass spectrometry method suitable for detecting the post-translational modification of interest.

Using the above-described method, the inventors purified cohesin (containing both Scc1 and SA2) from mitotic human cells, in which P1k1 was either active or in which P1k1 activity had been selectively inhibited with BI 2536.

To inhibit P1k1 activity, the specific P1k1 inhibitor BI 2536 was used, an ATP-competitive kinase inhibitor derived from a novel chemical series, the dihydropteridinones (WO 03/020722). The structure of BI 2536 is

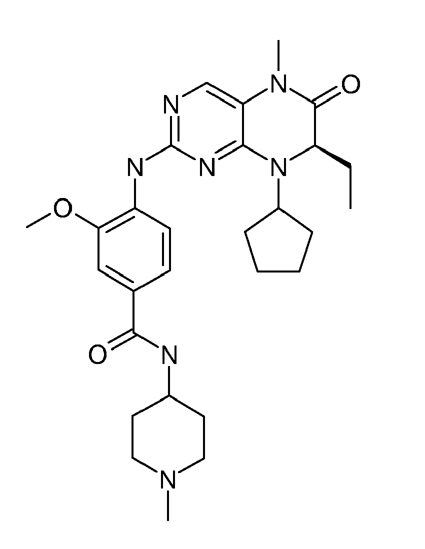

Next, phosphorylation sites were identified in both cohesin samples by mass spectrometry. A comparison of both datasets revealed that specific phosphorylation sites were missing on Scc1 and SA2 when cohesin had been purified from cells which had been treated with BI 2536, indicating that the phosphorylation of these sites must depend on P1k1 activity in vivo.

In summary, the experiments of the invention surprisingly revealed that the phosphorylation sites at serine 134 (or serine 138, respectively) and serine 545 of Scc1 (SEQ ID NO: 1) on the one hand and the phosphorylation sites at threonine 1160 (or serine 1161, respectively) and serine 1224 (or serine 1229, respectively) on SA2 (SEQ ID NO:2) on the other hand are sensitive to a P1k1 inhibitor, which means that phosphorylation of theses sites is P1k1-dependent; therefore, these sites are specific biomarkers for P1k1.

Next, the inventors confirmed that the BI 2536-sensitive phosphorylation sites listed above are indeed useful as biomarkers for P1k1 activity. To test this, antibodies were raised against a phosphorylated peptide that represents a fragment of SA2 which is phosphorylated on serine 1224; this peptide served as a representative of the peptides containing the P1k1 inhibitor-sensitive phospho sites listed above. Antibodies were purified from the resulting antisera that specifically recognize the SA2 peptide when it is phosphorylated on serine 1224, but not an otherwise identical SA2 peptide that is not phosphorylated on this amino acid residue. The inventors showed that these antibodies react specifically with SA2 in immunoblot experiments when SA2 is isolated from mitotic cells where SA2 is phosphorylated. In contrast, the antibodies do not react with SA2 when the protein is isolated from interphase cells where SA2 is not phosphorylated (Hauf et al., 2005), or when a mutant version of SA2 is isolated from miotic cells in which the 12 serine and threonine residues that were phosphorylated in mitosis in vivo, including serine 1224, had been mutated to alanine residues (called SA2-12xA; Hauf et al., 2005). These antibodies can also react with SA2 when interphase cohesin has been incubated in vitro with P1k1 and ATP. Together, these observations indicate that the antibodies react with SA2 only when SA2 has been phosphorylated on serine 1224.

To test if the anti-SA2 antibodies directed against a BI 2536-sensitive phospho site can be used to determine P1k1 activity in vivo, the reactivity of the antibodies with human cultured cells was analyzed in immunofluorescence microscopy experiments. The inventors observed that the antibodies did stain mitotic cells but did not react with cells in interphase, consistent with the possibility that the antibodies react only with the mitotically phosphorylated form of SA2 also in fixed human cells. This notion was further supported by the observation that the reactivity of the SA2 phospho-antibodies with mitotic cells was reduced when the levels of cohesin had been reduced in these cells by RNA interference (RNAi).

Importantly, the inventors showed that the reactivity of the SA2 phospho-antibodies with human mitotic cells was abolished when these cells had been treated with the P1k1 inhibitor BI 2536.

This result indicates that the anti-SA2 as well as the anti-Scc1 phospho-antibodies that bind to sites on which phosphorylation is sensitive to a P1k1 inhibitor can be used as biomarkers for P1k1 activity. This notion was also supported by experiments which showed that the reactivity of SA2 phospho-antibodies with human cells is also decreased when the levels of P1k1 had been reduced in these cells by RNAi. This observation shows that the ability of BI 2536 to inhibit the reactivity of SA2 phospho-antibodies with cells is caused by inhibition of P1k1, and not by inhibition of another unknown enzyme whose activity could also be affected by treatment with BI 2536.

The present invention provides proof of principle that the strategy described above for the phospho site at serine 1224 on SA2, can be used to develop biomarker assays for P1k1 activity. In analogy to serine 1224, phospho-specific antibodies can be raised against the other BI 2536 sensitive phosphorylation sites that have been identified on Scc1 and SA2.

In summary, the results obtained in the experiments of the invention show for the first time directly that the phosphorylation of specific amino acid residues in Scc1 and SA2 in human cells depends on P1k1 activity in vivo and that phospho-specific antibodies raised against the identified phosphorylation sites can be used for monitoring P1k1 activity in human cells.

Hence, based on the identification of phosphorylation sites of critical P1k1 targets, antibodies are provided that bind specifically to the phosphorylation sites that have been identified to be sensitive to a specific P1k1 inhibitor and that are useful in assays for assessing Scc1 and/or SA2 phosphorylation. Since phosphorylation is indicative of P1k1 activity, the antibodies are useful in assays to predict whether a tumor patient is likely to respond to a therapy with a P1k1 inhibitor and/or to test and monitor a compound's effect on P1k1 activity during therapy.

In a first aspect, the present invention provides an antibody which is specifically immunoreactive with a phosphorylation site on the cohesin subunit Scc1 (SEQ ID NO: 1) or on the cohesin subunit SA2 (SEQ ID NO:2), wherein phosphorylation of said site is dependent on P1k1 activity.

According to this aspect, the present invention relates to an antibody that binds to a phosphorylation site of the human cohesin subunit Scc1 (SEQ ID NO: 1) or of the human cohesin subunit SA2 (SEQ ID NO:2) when said phosphorylation site of Scc1 or SA2 is phosphorylated by P1k1 and that does not bind to said potential phosphorylation site when said phosphorylation site is not phosphorylated.

Specifically, the antibody of the invention is selected from a group of antibodies each of which binds to a phosphorylation site at
a) serine 134 (or serine 138, respectively) or serine 545 of Scc1 (SEQ ID NO:1) or
b) threonine 1160 (or serine 1161, respectively) or serine 1224 (or serine 1229, respectively) on SA2 (SEQ ID NO:2).

The term "antibodies" as used herein, refers to immunoglobulin molecules and immunologically active portions of immunoglobulin molecules, i.e., molecules that contain an antigen binding site or paratope. Examples of such antibody molecules are intact immunoglobulin molecules, substantially intact immunoglobulin molecules and those portions of an immunoglobulin molecule that contain the paratope, including those portions known in the art as Fab, Fab', F(ab')₂ and F(v).

The antibody of the invention may be a polyclonal or monoclonal antibody, preferably, it is monoclonal.

As stated above, an antibody of the invention immunoreacts with an epitopic site of phosphorylated Scc1 or SA2. The term "epitopic site" refers to an antigenic determinant that consists of chemically active surface groupings of molecules such as amino acids or sugar side chains that usually have specific three dimensional structural characteristics, as well as specific charge characteristics. The preferred peptides containing an ectopic site and thus used for immunization to obtain the antibodies of the invention are Scc1 or SA2 peptide fragments consisting essentially of about 9 to 21, preferably about 11 to about 17 amino acid residues, wherein about 4 to 10, preferably about 5 to about 8 amino acid residues are positioned on each side of the phosphorylation site.

For generation of the antibodies of the invention, standard procedures for generating antibodies, in particular phospho-specific antibodies, can be used (Wisdom, 1994).

To obtain phospho-specific anti-Scc1 and/or SA2 antibodies, a series of epitopic Scc1 and/or SA2 peptide fragments containing the respective phosphorylation sites of Scc1 and/or SA2, respectively, with a minimum length of six amino acids, usually about 9 to 21, preferably of about 11 to 17 amino acids, are synthesized.

The peptides may be synthesized such that they have a cysteine attached at the C-terminus to permit the unidirectional attachment to a carrier protein through a connecting bridge. Standard methods of conjugation also include the glutaraldehyde method which couples primarily through the α-amino group and ε-amino group, and the Maleimidobenzoyl-N-hydroxysuccinimide ester (MBS) method which couples through sulfhydryl groups. The carrier proteins most commonly used are Keyhole Limpet Hemocyanin (KLH), Bovine Serum Albumin (BSA), Rabbit Serum Albumin (RSA), Ovalbumin or Thyroglobulin (THY).

Synthesis of the phosphorylated peptides and, for specificity control and for affinity purification in the case of polyclonal antibodies, of unphosphorylated peptides, can be done according to standard procedures as described by Wisdom, 1994, e.g. by the standard Merrifield (Boc) solid phase synthesis. Methods suitable for synthesizing the phospho-peptides for immunization and of the ultimately resulting antibodies of the invention are also described by Wisdom, 1994, and inter alia, for serine-phosphorylated antibodies by Lewis at al., 1994, and for tyrosine-phosphorylated antibodies in US 5,599,681.

Another method suitable for generating the antibodies of the invention is described in US 6,309,863, which suggests a method for producing polyclonal or monoclonal phospho-specific antibodies by using for immunization a peptide containing a phospho-peptide mimetic.

The invention also provides an immunogenic composition comprising an antigenic phospho-peptide derived from a polypeptide selected from SEQ ID NO:1 or SEQ ID NO:2, as described above, wherein said phospho-peptide.

For generating polyclonal antibodies, animals, usually rabbits, e.g. New Zealand White rabbits, are immunized with the synthetic phospho-peptide (conjugate). IgG from best responding animals, as determined by enzyme-linked immunosorbent assay against the phospho-peptide and corresponding unphosphorylated peptide, is purified by standard methods, e.g. as described in Rena et al., 1999, for the generation of generation of phosphospecific antibodies for FKHR (Forkhead transcription factors FOXO1). In brief, by this method antibodies reactive with the unphosphorylated peptide are removed by adsorption using an affinity column loaded with the corresponding unphosphorylated peptide, followed by affinity chromatography, e.g. on CH-Sepharose to which the corresponding phospho-peptide antigen had been covalently attached, and elution of the phospho-specific antibodies.

Preferably, the antibodies of the invention are monoclonal antibodies that bind specifically to the predetermined phospho-protein epitope. Monoclonal antibody secreting hybridomas are produced using the phospho-peptides in conventional techniques (see e.g., Harlow and Lane, 1988). Phospho-specific monoclonal antibodies are usually produced by murine hybridomas formed by fusion of:
a) a mouse myeloma or hybridoma which does not secrete antibody with,
b) a murine spleen cell which secretes antibodies, obtained from a mouse immunized with a mimetic-containing polypeptide antigen.

Typically, several mice are immunized with a primary injection of antigen followed by a number of boosting injections. During or after the immunization procedure, sera from the mice are screened to identify mice which have mounted a substantial immune response. For selected mice, the spleen cells are obtained and fusions are performed. Suitable fusion techniques include, for example, the Sendai virus technique (Kohler and Milstein, 1975), or the polyethylene glycol method (Kennet, 1980).

The resulting hybridomas are then screened for production of antibodies specific for the antigen. Suitable screening technique are a solid phase radioimmunoassay or ELISA techniques, e.g. coating 96-well plates with phospho- or nonphosphopeptides, incubating with hybridoma supernatant and then using a labelled secondary antibody, e.g. a horseradish peroxidase-conjugated antibody, for detection. Using these technique, a solid phase is prepared by coupling the appropriate antigen to an insoluble matrix. The immunoadsorbent is brought into contact with culture supernatants of hybridomas. After a period of incubation, the solid phase is separated from the supernatants, then contacted with a labelled antibody against murine immunoglobulin. Label associated with the immunoadsorbent indicates the presence of hybridoma products reactive to the antigen.

According to traditional methods, the monoclonal antibodies are produced in large quantities by injecting antibody producing hybridomas into the peritoneal cavity of mice and after an appropriate time, harvesting ascitic fluid from the mice. The thus obtained monoclonal antibodies are then isolated from the fluid.

Preferably, alternative methods are used by which the hybridomas are cultured *in vitro*; such methods have been recently developed, mainly due to animal-welfare issues:
The simplest approach for producing monoclonal antibodies *in vitro* is to grow the hybridoma cultures in batches and purify the monoclonal antibodies from the culture medium. Serum-free media specifically formulated to support the growth of hybridoma cell lines are commercially available. After adaptation of the cells to serum-free media, cell cultures are allowed to incubate in commonly used tissue-culture flasks under standard growth conditions; the monoclonal antibody is then harvested from the medium.

To increase cell viability and the density at which the cells grow and thus increase monoclonal antibody concentration, methods can be used that employ spinner flasks or roller bottles, alternatively, commercially available gas-permeable bags may be used. To permit cells to grow at high densities, the use of a barrier, either a hollow fiber or a membrane, with a low-molecular-weight cutoff has been implemented in several devices for in *vitro culture* of hybridoma. In these semipermeable-membrane-based systems, the cells are isolated and the monoclonal antibodies produced in a small chamber separated by a barrier from a larger compartment that contains the culture media. This compartmentalization makes it possible to achieve monoclonal antibody concentrations comparable with those in mouse ascites. In a hollow-fiber bioreactor, the medium is continuously pumped through a circuit that consists of a hollow-fiber cartridge, gas-permeable tubing that oxygenates the media, and a medium reservoir. The hollow-fiber cartridge is composed of semipermeable multiple fibers that run through a chamber that contains hybridoma cells growing at high density. Such hollow-fiber bioreactors are commercially available, e.g. the so-called "Tecnomouse" from Integra Biosciences).

Next, the resulting antibodies are, using standard technology (e.g. Wisdom, 1994), purified and, in order to determine the extent of their phosphospecificity, characterized by immunoassays, e.g. enzyme-linked immunosorbent assay (ELISA), for binding to the respective phospho- and non-phosphopeptides. To this end phosphorylated and non-phosphorylated peptides are coated onto 96-well microtiter plates and, after blocking unspecific binding sites with excess protein, incubated with the solution containing the antibodies to be characterized (e.g. hybridoma supernatant, dilutions of purified antibodies). After allowing the antibodies to bind, unbound antibodies are washed off and a secondary antibody directed towards either mouse IgG or rabbit IgG is added to the wells. The secondary antibody is typically conjugated with an enzyme such as horse radish peroxidase (HRP) in order to visualize the specific binding of primary antibody to the coated peptide by the addition of a suitable substrate (e.g. 2,2'-Azino-bis(3-ethylbenzothiazoline-6-sulfonic acid) diammonium salt from Sigma Aldrich)

Antibodies specific for a given phospho-epitope should recognize the phosphorylated but not the non-phosphorylated form of the peptide used.

Subsequently, the antibodies of the invention are analysed by standard Western blotting technique to further determine specificity against Scc1 or SA2, respectively. To this end, whole cell lysates from human tumor cell lines are separated by SDS-PAGE and blotted onto e.g. nitrocellulose membranes. The membrane is subsequently incubated with blocking solution and then the antibody directed against the phosphoepitope(s) is added. After incubation, unbound antibody is washed away and a secondary antibody conjugated e.g. with HRP (horse raddish peroxidase) is added. After washing off unbound antibodies the bound antibodies are visualized by ECL (enhanced chemiluminesce). The antibodies should give a single band at the molecular weight corresponding to Scc1 or SA2. To check the specificity of the antibodies, incubation of the membrane with the primary (e.g. phosphospecific) antibody is also done in the presence of phospho- or nonphospho- polypeptide selected from SEQ ID NO:1 or SEQ ID NO:2, as described above. The obtained signal is considered to be specific if it can be competed away with the phospho-polypeptide but not with the corresponding non-phospho-polypeptide.

For the sites that have not been unambiguously identified as phospho-sites (as e.g. in the case of serine 138 or serine 134, respectively, on Scc1), two antibodies are raised against peptides which are each phosphorylated on only one of the sites in question. The obtained antibodies, each one immunoreactive against only one of the phosphorylation sites in question, are tested for reactivity on the substrate (e.g. Scc1) in its phosphorylated state (e.g. isolated from mitotic cells). The site that is recognized by one antibody (or possibly both sites are recognised each by one antibody in the case that both sites are P1k1-dependent phospho-sites) can be confirmed as the relevant P1k1 biomarker. In the case that both sites are P1k1-dependent, a monoclonal antibody can be generated that recognizes both sites.

As shown in the the experiments of the invention, antibodies can be selected that distinguish between mitotically arrested and logarithmically growing cells. To this end, a lysate of mitotically arrested cells (in which Scc1 and SA2 are predominantly present in their phosphorylated state due to P1k1 activity) is contacted with a candidate antibody and the reactivity of the antibody is determined by Western blot analysis as described above. The obtained signal is compared side by side with the signal obtained with lysates of logarithmically growing cells. Antibodies which yield a strong signal in lysates from mitotically arrested cells but do only yield a very weak signal in lysates from logarithmically growing cells are best suited to be used in assays for assessing P1k1 activity.

The specificity of the antibodies of the invention can be further evaluated by immunohistochemistry in tissue sections from human tumors. For this purpose, xenografts from different tumor types are prepared, the xenografted mice are treated with BI 2536 or another P1k1 inhibitor, using the dose and schedule necessary to achieve a tumor regression. The mice are sacrificed, the tumors excised and processed for immunohistochemical analysis.

In addition, clinical samples from human tumors are analyzed in a similar manner, using the assay conditions established on the xenograft studies: Frozen and paraffin-embedded sections are prepared and analysed in an immunofluorescence assay using the antibodies of the invention, e.g. using an indirect immunofluorescence assay and the avidin-biotin (ABC) immunoperoxidase method described e.g. by. Garin-Chesa et al. 1990. To confirm the specific binding of the antibodies to mitotic cells, double labeling experiments with the mitosis marker phospho-histone- H3 antibody (Upstate) are carried out.

In a further aspect, the invention also provides a method for detecting the presence of Scc1 and/or SA2 that are phosphorylated in a P1k1-dependent manner, wherein a biological sample suspected of P1k1 activity (and thus containing Scc1 and/or SA2 phosphorylated at the relevant sites) is contacted with a phospho-specific antibody of the invention and binding of the antibody is determined. Due to the antibody's ability to distinguish between phosphorylated and non-phosphorylated Scc1 or SA2, which are substrates of Polo-like kinase 1 (P1k1), the readout of said measurement can be directly correlated with P1k1 activity. In an embodiment of the invention, in order to determine the pharmacodynamics of a drug that acts as a P1k1 inhibitor, i.e. the action of the drug in the body over a period of time, including the processes of absorption, distribution, localisation in the tissues, biotransformation, and excretion, the activity of P1k1 is determined in a biological sample. For determining the pharmacodynamic activity of a drug that acts as a P1k1 inhibitor, the biological sample is derived from a patient who is being treated with said P1k1 inhibitor.

The sample may be any tissue or body fluid from a subject. Ideally, the sample comprises proliferating cells that are positive for cohesin, e.g. tumor cells or, alternatively, cells derived from tissue with mitotic activity, e. g. hair root cells (radix pili cells) or mouth mucosa cells.

In a further aspect, the invention relates to a method for determining a compound's effect on P1k1 activity in an individual treated with a compound by determining *ex vivo* before and after administration of said compound to said individual the presence of phosphorylated Scc1 and/or SA2 in a sample and comparing the levels of phosphorylated Scc1 and/or SA2 before and after administration of the compound, wherein a reduced level of phosphorylated Scc1 and/or SA2 at P1K1-dependent sites upon administration of the compound is indicative of its inhibitory action on P1k1.

In a variation of this method, the compound's effect on P1k1 activity may be determined over a period of time at pre-defined time points by comparing the levels of phosphorylated Scc1 and/or SA2 over said period of time, wherein a reduced level of phosphorylated Scc1 and/or SA2 at P1K1-dependent sites is indicative of a compound's therapeutic effect.

In principle, the above described methods can be conducted by using a single antibody of the invention, e.g. an anti-SA2 antibody, however, two or more assays employing different phospho-specific anti-SA2 and/or anti-Scc1 antibodies may be done in parallel.

In the above described assays, the desired therapeutic effect of a P1k1 inhibitor should manifest itself in a decrease of phosphorylated Scc1/SA2 at P1K1-dependent sites relative to the overall amount of Scc1/SA2.

The antibodies of the invention are also useful for determining the specificity of a compound's inhibitory effect on P1k1. To this end, the amount of phosphorylated Scc1/SA2 is compared with the level of other cell cycle biomarkers, e.g. with the level of cyclinB, phosphorylated histone H3 or MPM2-reactive signal (or other enzymes or markers expressed in mitosis). Administration of a specific P1k1 inhibitor should result in a decrease of the P1k1-specific Scc1/SA2 phosphorylation signal, while the other cell cycle markers should be unaffected by the compound. Thus, upon treatment of a patient with P1k1 inhibitor the biological sample from a tissue or fluid that normally contains proliferating cells should, at a given time point, show high levels of mitotic markers (e.g. cyclin B, MPM-2 or histon H3 phospho-signal, Ki-67, Aurora kinase etc.) and reduced or no levels of P1k1-specific Scc1/SA2 phosphorylation signal. The degree of the observed mitotic arrest and the degree of down-regulation of P1k1-specific Scc1/SA2 phosphorylation signal can be used to assess the pharmacodynamic activity of the administered drug. In insights gained from this evaluation can be used to help guiding the adjustment of drug dose and treatment schedule.

The antibodies of the invention may also be used for selecting patients for treatment with P1k1 inhibitors. To this end, tumor samples from cancer patients are tested for P1k1 activity as described above. Since a high level of Scc1/SA2 phosphorylation at the relevant sites in the tumors of patients may be a consequence of aberrant P1k1 activity, it may be expected that these patients are likely to benefit from treatment with a P1k1 inhibitor.

In a further aspect, the invention relates to a method for treating a patient or a patient population having a tumor that correlates with P1k1 activity, comprising the steps of
a) determining whether the tumor of said patient contains one or more P1k1-dependent phosphorylations,
b) selecting a patient or patient population whose tumor has one or more P1k1-dependent phosphorylation(s),
c) administering to said patient a therapeutically effective amount of a P1k1 inhibitor.

In a preferred embodiment, step b) comprises determining one or more P1k1-dependent phosphorylations on the cohesin subunits Scc1 (SEQ ID NO: 1) and/or SA2 (SEQ ID NO:2),

Specifically, step b) comprises determining one or more P1k1-dependent phosphorylations
i) at serine 134 (or serine 138, respectively) or serine 545 of Scc1 (SEQ ID NO:1) or
ii) at threonine 1160 (or serine 1161, respectively) or serine 1224 (or serine 1229, respectively) on SA2 (SEQ ID NO:2).

The P1k1 inhibitor administered in step c) may be BI 2536 or any other therapeutically effective dihydropteridinone compound disclosed in WO 03/020722 or WO 04/076454. Also, other therapeutically active compounds described as P1k1 inhibitors, e.g. in WO 06/063806, WO 05/042505, WO 03/093249, WO 04/043936 and WO 05/019193, may be used.

Alternatively, the P1k1 inhibitor may be an anti-Plk1 si-RNA or antisense molecule as described in WO 03/070283.

Since P1k1 is a kinase that has crucial role in regulating multiple steps in mitosis, the method of the invention is useful for multiple cancer types in a broad indication spectrum, i.e. both solid tumors and hematological malignancies. Examples are non-small-cell lung cancer (NSCLC), breast cancer, prostate cancer, pancreatic adenocarcinoma, B-NHL, colon cancer, acute myeloid leukemia, cancer of the head and neck.

In a further aspect, the invention relates to a method of advertising and selling a P1k1 inhibitor for the treatment of a patient or patient population in need of such treatment, wherein such need is defined by the presence of or more P1k1-dependent phosphorylations in the tumor of said patient or patient population
iii) at serine 134 (or serine 138, respectively) or serine 545 of Scc1 (SEQ ID NO:1) or
iv) at threonine 1160 (or serine 1161, respectively) or serine 1224 (or serine 1229, respectively) of SA2 (SEQ ID NO:2).

The methods of the invention for determining Scc1 and/or SA2 phosphorylation status can be carried out using standard immunohistochemical methods. To this end the collected patient sample is prepared for immonohistochemical analysis as it has been described previously (e.g by Harlow and Lane, supra). Briefly, the patient sample containing proliferating cells is embedded into an appropriate matrix (e.g. paraffin or TissueTec for cryopreservation) or smears or spreads are prepared from collected cellular material. Sections from the embedded tissues are generated and are subjected to primary and secondary antibody incubations. P1k1-specific Scc1/SA2 phosphorylation signal or proliferation markers are visualized using peroxidase staining or an equivalent technique. In addition, standard tissue staining techniques such as hematoxilin staining can be used for histological analysis (Harlow and Lane, supra). Instead of using the antibodies of present invention for immunohistochemical analysis, the antibodies may be also used for the analysis of the patient sample by Western Blot (as described above) or isolated patient cells can be subjected to FACS analysis. To this end, such isolated cells are fixed, permeabilized and incubated with primary (e.g. Scc1/SA2 phosphorylation specific antibodies and antibodies specific for other mitosis markers as described above) and secondary antibodies conjugated with fluorescent tags. The signals for Scc1/SA2 phosphorylation status and the signal for other mitotic markers is then determined by flow cytometry in a fluorescensce activated cell sorter (FACS).

In another aspect, the antibodies of present invention are useful in screening methods for determining or confirming a compound's ability to inhibit P1k1 in a cell.

Such screening methods can be conducted according to known principles, e.g. by adapting the method described in US 6,287,784 that has been suggested for screening kinase inhibitors by determining autophosphorylation of kinase receptors. In an assay for identifying P1k1 inhibitors, by way of example, a first solid phase is coated with a substantially homogeneous population of cells having P1k1 activity, e.g. proliferating cells that are arrested/synchronized in mitosis (e.g. by incubating them with nocodazole), so that the cells adhere to the first solid phase. Following exposure to the candidate compounds for a period of time sufficient for the compound to exert its inhibitory effect, e.g. for 5 minutes to 5 hours, the adherent cells are solubilized, thereby releasing cell lysate. A second solid phase is coated with a capture antibody that binds to Scc 1 or SA2 at a site different from a phosphorylation site. The cell lysate obtained from the first solid support is added to the second solid support containing the adhering capture antibody in order to capture Scc1 or SA2, respectively. A washing step is then carried out to remove unbound cell lysate, leaving the captured substrate bound to the second solid support. Next, the captured Scc1 or SA2 is exposed to a labelled antibody of the invention, which identifies a phosphorylated residue in Scc 1 or SA2. In a final step, binding of the phospho-antibody to the captured substrate is measured. The obtained signal is compared with the signal obtained from control cells without exposure of compound.

### Brief description of the Figures:

- Figure 1:: Summary of phosphorylation sites detected on Scc1 and SA2 by mass spectrometry.
- Figure 2:: Western blot of the antibody pSA2 (recognizing S1224 of SA2) showing that it detects a mitosis specific, BI 2536 sensitive and phosphorylation specific signal.
- Figure 3:: Immunofluorescence staining showing that pSA2 detects only mitotic cell stages in a BI 2536 sensitive manner.
- Figure 4:: Depletion of P1k1 by siRNA followed by immunofluorescence staining with pSA2 confirms that the signal is P1k1 dependent.
- Figure 5:: *in vitro* phoshporylation of immunopurified interphase SA2 by P1k1 shows that pSA2 recognises a phosphorylation site which is generated directly by P1k1 and is also BI 2536 sensitive *in vitro*

In the following Examples, if not otherwise stated, the following materials and methods are used:

### Cell lines, growth conditions and siRNA experiments

HeLa cells expressing 9myc-tagged wild type and mutant human SA2 are described previously (Hauf et al., 2005). Untransfected HeLa cells are grown in DMEM supplemented with 10 % FCS, 0.2 mM L-glutamine, 100 U/ml penicillin, and 100 µg/ml streptomycin. For growth of stably transfected HeLa tet-on cells, the medium is supplemented with 200 µg/ml G418 and 100 µg/ml Hygromycin. Hydroxyurea, Nocodazole and BI 2536 are used at a concentration of 2 mM, 330 nM and 64 nM respectively.

RNAi: siRNA oligos are synthesized by Ambion. Sense sequences of siRNA oligonucleotides used are: SA2: from position 778-797 of the SA2 coding sequence (AL355476); P1k1 : from position 889 to 910 of the P1k1 coding sequence (NM 00530). siRNA-transfection was performed as described (Hirota et al. 2004) at a concentration of 100 nM (P1k1) or 150 nM (SA2).

### Antibodies

To detect myc-tagged protein, monoclonal 9E10 mouse-anti-myc antibody (Waizenegger et al., 2000) is used. Polyclonal antibodies to human SA2 (designated "446") have been described (Sumara et al., 2000). For immunofluorescence the mouse - anti phospho-Ser10 on histone H3 antibodies (H3S10ph) is from Cell Signalling and used according to manufacturers instructions, the goat - anti SA2 antibodies are from Bethyl labs and used according to manufacturers instructions.

### Mass Spectrometry

Immunopurified SA2-cohesin complex is digested in solution with different proteases (trypsin, Glu-C, chymotrypsin) according to Yates et al., 2000. Proteolytic peptides are applied to a precolumn (PepMAP C18, 0.3 x 5 mm, Dionex) and eluted onto an analytical column (PepMAP C18, 75 µm x 150 mm, Dionex). The eluted peptides are introduced via a nanospray ion source interface (Proxeon) into either an ion trap mass spectrometer (Finnigan LTQ) or an ion trap mass spectrometer coupled to a fourier transform ion cyclotron resonance mass detector (FT-ICR). The mass spectrometer cycles through seven scans, one full mass scan followed by six tandem mass scans of the six most intense ions. Sequenced peptides are put onto an exclusion list for one minute. All tandem mass spectra are searched against the human non-redundant protein database by using algorithms included in Bioworks 3.2 (Thermo Finnigan) and MASCOT 2.1 (Matrix Science). Any phosphopeptide matched by computer searching algorithms is verified manually.

### Immunoprecipitation

SA2-Cohesin is purified by peptide antibody affinity chromatography using the 446 antibody (Sumara et al. 2000). 300 to 500 µg of purified antibody are coupled and crosslinked to Affi-prep protein A beads (BioRad) at 1 µg antibody/µl beads. Cohesin was immunoprecipitated from 6 to 10 ml HeLa extract containing approximately 10 µg protein/µl. Extraction buffer for BI 2536 - treated cells additionally contained 64 nM BI 2536.

### In vitro phosphorylation assay

SA2-cohesin complex is purified by immunoprecipitation with affinity-purified SA2 antibody (Sumara I et al, 2000). In vitro phosphorylation is performed as described (Kraft et al., 2003) using 10 mM ATP and recombinant 6xHis-tagged P1k1. Recombinant human P1k1 is expressed as a 6xHis-tagged fusion protein using a baculoviral expression system and purified by affinity chromatography using Ni-NTA (QIAGEN). Where indicated, reactions are supplemented with 1 µM BI 2536.

### Immunofluorescence

For immunofluorescence, cells are grown on coverslips and treated with 64 nM BI 2536 45 minutes prior to fixation where indicated. Fixation, immunostaining, and image acquisition are performed as described (Waizenegger et al., 2000).

### Example 1

Identification of Mitosis-Specific Phosphorylation Sites on Human SA2 and Scc1 Whose Generation in Vivo Depends on P1k1 Activity

In vertebrate cells, including human cells, the cohesin complex dissociates from chromosome arms during prophase and prometaphase (Losada et al., 1998; Sumara et al., 2000; Waizenegger et al., 2000). Previous studies have shown that this process depends on P1k1 (Losada et al., 2002; Sumara et al., 2002; Gimenez-Abian et al., 2004) and on phosphorylation of the cohesin subunit SA2 (Hauf et al., 2005). It was assumed that the phosphorylation of SA2 is mediated by P1k1 because both depletion of P1k1 by RNA interference (Gimenez-Abian et al., 2004) and expression of a non-phosphorylatable SA2 mutant (Hauf et al., 2005) interfere with dissociation of cohesin from chromosome arms in human cells, and because P1k1 can phosphorylate SA2 in vitro (Losada et aal., 2002; Sumara et al., 2002). While it was shown that Scc1 is phosphorylated in mitosis (Hauf et al., 2005) its phosphorylation in vivo is not essential for mitotic progression (Hauf et al., 2005).

However, Scc1 could be phosphorylated in vitro by P1k1 and this phosphorylation was shown to enhance its cleavage by Separase. Since there was no direct evidence that the phosphorylation of SA2 or Scc1 depends on P1k1 in vivo the inventors performed mass spectrometry experiments to identify which of the previously identified phosphorylation sites on SA2 and Scc1 (Hauf et al., 2005) can not be generated when P1k1 is inhibited.

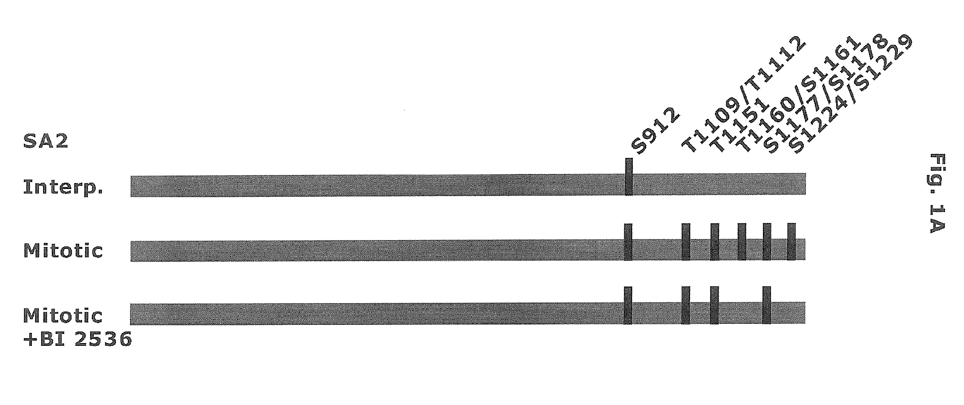

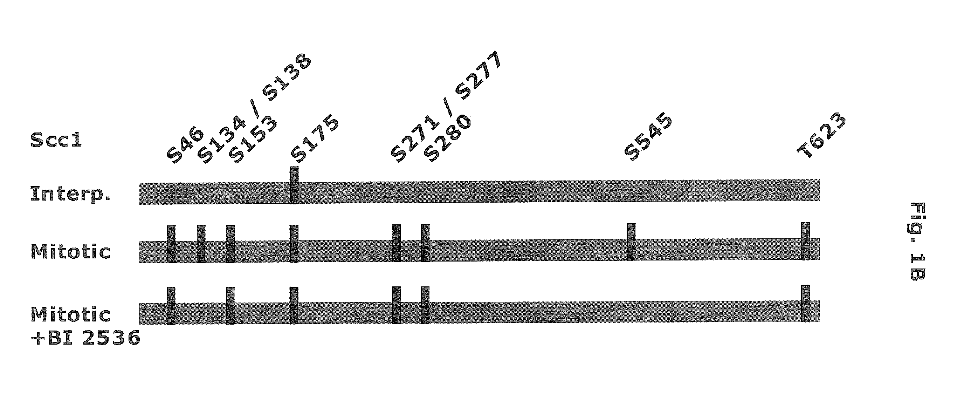

To achieve this goal, human HeLa cells are arrested in mitosis by treatment with nocodazole and then either the cells are treated for 2 hours with the P1k1 inhibitor BI 2536 or left them untreated. Subsequently, mitotic cells are harvested by shake off, cell lysates are prepared, and cohesin complexes containing SA2 and Scc1 are purified from these by immunoprecipitation with antibodies to SA2 (designated "446"; Sumara et al. 2000). Purified proteins are digested with various proteases in solution and analysed by HPLC-ESI-MS/MS. In total, 6 phosphorylation sites are identified on SA2. Comparison of these sites with sites that are identified in parallel on SA2 purified from interphase HeLa cells shows that 5 of the sites are mitosis-specific (Figure 1A). Importantly, two of the mitosis-specific sites could never be found on SA2 when the protein had been isolated from cells treated with BI 2536, indicating that the generation of these two phosphorylation sites on SA2 depends on P1k1 in vivo. On Scc1, 8 phosphorylation sites are identified in total. Comparison of these sites with sites that are identified in parallel on Scc1 purified from interphase HeLa cells shows that 7 of the sites are mitosis-specific (Figure 1B). Importantly, two of the mitosis-specific sites could never be found on Scc1 when the protein had been isolated from cells treated with BI 2536, indicating that the generation of these two phosphorylation sites on Scc1 depends on P1k1 in vivo.

In the experiments the inventors could not detect all of the phosphorylation sites on SA2 and Scc1 that have previously been described (Hauf et al. 2005). This difference could be due to the fact that the purification and the mass spectrometry experiments are carried out with a protocol and on a mass spectrometer (the LTQ from Thermo Finnigan) that are different than the protocol and instrument (the LCQ from Thermo Finnigan) used in the previous study.

### Figure 1:

A) The schematic representation shows the phosphorylation sites detected on SA2 from interphase cells (interp.), mitotic cells and from mitotic cells treated with BI 2536.
B) The schematic representation shows the phosphorylation sites detected on Scc1 from interphase cells (interp.), mitotic cells and from mitotic cells treated with BI 2536.

### Example 2

### Generation of a Phospho-Specific Antibody to SA2

### a) Generation of Antiserum Against the P1k1 Dependent Phospho-Epitope S1224 on SA2

To generate antibodies that specifically react with a BI 2536 sensitive mitotic phosphorylation site on SA2, rabbits are immunized with a synthetic phosphopeptide. This peptide represents the part of the SA2 amino acid sequence which is found by mass spectrometry to be phosphorylated in human cultured cells specifically in mitosis and which is sensitive to BI 2536 (Figure 1). The peptide is designated 1746 (corresponding to amino acid residues 1221 to 1228 of human SA2 (SEQ ID NO:2), phosphorylated at serine 1224 (a carboxy-terminal cysteine residue is added to allow subsequent coupling of the peptides to a carrier protein that is not derived from the SA2 sequence)

The antibodies generated are designated "1746 antibodies" or short "pSA2".

The synthetic peptide is coupled via the carboxy-terminal cysteine residue to keyhole limpet hemocyanin (KLH; Merck Biosciences, Nottingham, UK) following the manufacturer's instructions. 3 mg of lyophilized peptide are dissolved in 700 µl of buffer containing 3 M guanidine hydrochloride and 0.5 M sodium tetraborate, pH 6.9, and the solution is added to 300 µl of the same buffer in which 3 mg KLH had been dissolved. After removal of oxygen by aerating with argon, the reaction mixture is incubated for 1 hour at room temperature and subsequently stored at 4 °C. To determine the efficiency of the coupling reaction, 10 µl aliquots of the peptide-KLH solution are removed at the beginning and the end of the reaction, mixed with 50 µl of Ellman's reagent (50 mM sodium acetate, 2 mM 5,5'-Dithiobis(2-nitrobenzoic acid); Sigma-Aldrich, St.Louis, MO), 100 µl solution containing 1 M Tris-HCl, pH 8.0 and 840 µl water, and the absorbance of the samples is measured at 412-nm ("Ellman test"). A decrease in absorbance indicated a reduction in the amount of free thiol groups and is a measure for the efficiency of peptide coupling to KLH (Ellman, 1959; Bulaj et al., 1998). The coupled peptides are used to immunize rabbits at a commercial facility (Gramsch Laboratories, Schwabhausen, Germany). The resulting sera are frozen in 10 ml aliquots in liquid nitrogen and stored at -80 °C.

### b) Generation of Peptide Affinity Columns for the Purification of the Phospho-Specific SA2 Antibodies

To purify phospho-specific antibodies from the rabbit serum, peptide affinity columns are generated. The SA2 phospho-peptide described above is coupled to a chromatography resin, and the resulting column is then used to bind those antibodies that specifically react with the phospho-peptide. In addition, a peptide is synthesized that is identical in sequence to the sequence of the phospho-peptide, except that it contains an unmodified serine residue instead of the phospho-serine residue. This peptide is also coupled to a chromatography resin, and the resulting column is used to remove antibodies from the rabbit sera that react with the SA2 peptide independently of its phosphorylation state.

To generate the peptide affinity column, Poros beads (Applied Biosystems, Foster City, CA) are activated with the bi-functional cross-linker sulfo-GMBS (Pierce, Rockford, IL) according to the manufacturer's instructions, which result in the generation of maleimid groups. Of the synthetic peptide, 5 mg of lyophilized peptide are dissolved in 3 ml buffer containing 3 M guanidine hydrochloride and 0.1 M Hepes-KOH, pH 7.9, yielding a peptide concentration of~2.27 µmol. 310 mg of activated Poros beads are mixed with the peptide solution and the suspension is incubated on a rotary shaker for one hour at room temperature. Subsequently, the Poros beads are collected by low speed centrifugation and the coupling reaction is stopped by washing with at least 10 bead volumes of a buffer containing 150 mM NaCl, 20 mM Tris-HCl, pH 7.5. The efficiency of cross-linking is determined by the Ellman test (see a). Resin coupled to peptide is stored in batch at 4°C. To obtain a homogenously packed affinity column the coupled resin is transferred into a HPLC column (0.7 ml; Applied Biosystems) by employing a filling device (Applied Biosystems) that is connected to a BIOCAD HPLC system (Applied Biosystems).

### c) Purification and Concentration of the Phospho-Specific Antibodies to SA2

Phospho-specific antibodies are purified from the serum generated as described under a) by the following strategy: Antibodies that react with the SA2 peptide independently of its phosphorylation state are first removed from each serum by passing it over the affinity column that contained the corresponding antigenic peptide in its non-phosphorylated form. Subsequently the serum is passed over a second affinity column which contained the phosphorylated antigenic peptide. Both columns are connected in line to a BIOCAD HPLC and are equilibrated with 20 column volumes (cv) of HBS buffer (20 mM Hepes-KOH, 150 mM NaCl) at a flow rate of 7 ml/min. All chromatography steps are carried out at room temperature and are monitored by measuring the absorbance on-line at 280 nm. Aliquots of antiserum are rapidly thawed in a water bath at 37 °C. The thawed serum is extracted once for two minutes with an equal volume of chloroform to remove lipids. To separate the organic fraction from the serum, the emulsion was centrifuged at 2,000 rpm in a Varifuge 3.0R (Heraeus; Kendro, Asheville, NC) for 15 minutes at 4 °C. Subsequently, the serum is filtered through a 0.45 µm syringe filter (MILLEX-HA; MILLIPORE, Billerica, MA) and stored on ice. Antibodies are purified from 20 ml of serum by four subsequent chromatography runs in each of which 5 ml aliquots are processed. 5 ml of chloroform extracted serum are loaded onto the column with a flow rate of 5 ml/min (column pressure: 20-25 bar). The column is washed with 50 cv of HBS.

Subsequently the two columns are disconnected and bound proteins are eluted separately from the two columns. Bound proteins are eluted first with Buffer A (1.5 M MgCl₂, 100 mM sodium acetate pH 5.2) and subsequently by Buffer B (100 mM glycine-HCl, pH 2.45). The pH of the eluted fractions is immediately neutralized by the addition of 1/10 volume of 2 M Hepes-KOH, pH 7.9. The fractions obtained by elution with Buffer A or with Buffer B are kept separate during all subsequent steps because antibodies eluted by high MgCl₂ concentrations (Buffer A) or by low pH (Buffer B) can differ in their abilities to recognise their antigen under different conditions. The peak fractions are dialyzed over-night at 4 °C against 2 liters of buffer C (20 mM Hepes-KOH, 150 mM NaCl, 10% glycerol), using dialysis tubing with a molecular weight cut-off (MWCO) of 3,500 Da (Spectra/Por; Spectrum Laboratories, Rancho Dominguez, CA). Buffer C is exchanged once after 3 hours. Subsequently, the antibodies are concentrated by ultra-filtration in a Centriprep centrifugal filter device (YM-30, MWCO 30 kDA, 15 ml, Amicon; MILLIPORE) at 3,000 rpm (Varifuge 3.0R) and 4°C. The final protein concentration is determined by photometric measurements at a wavelength of 280 nm. An OD₂₈₀ = 1 of a mixture of IgG and IgM corresponds to 1.4 mg/ml protein (Harlow and Lane, 1988) purity of the samples is assessed by SDS-PAGE and silver staining, and 100 µl aliquots of the purified antibodies are frozen in liquid nitrogen and stored at -80°C. A typical purification yields 6 to 9 mg of pure antibodies from 20 ml of antiserum.

### d) Characterization of the Specificity of the Phospho- SA2 Antibodies

The following experiments are performed to determine if the antibodies that are eluted from the affinity column containing phosphorylated antigenic peptide are specifically reacting with a BI 2536 sensitive phosphorylation on SA2.

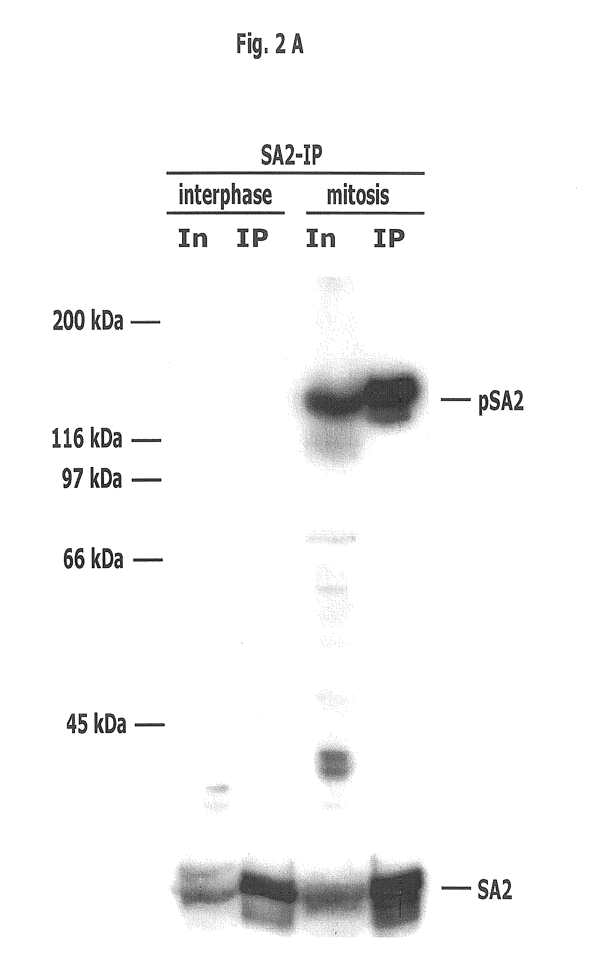

First, cohesin is isolated by immunoprecipitation with the 446 antibodies from cultured human HeLa cells that are either in interphase or are arrested in mitosis by treatment with nocodazole. Proteins in the immunoprecipitates as well as the low speed supernatant of the cell extracts are separated by SDS-PAGE and analysed by immunoblotting with the 1746 antibodies. For this purpose, proteins separated by gel electrophoresis are transferred to a PVDF blotting membrane and incubated with the antibodies purified as described under b. The reactivity of the antibodies with proteins bound to the PDVF membrane is detected by incubation with enzyme-linked secondary antibodies that can be detected by enhanced chemoluminescence (ECL). As is shown in Figure 2A, 1746 antibodies only react with SA2 that has been isolated from mitotic cells where SA2 is phosphorylated, whereas the antibodies do not react with SA2 from interphase cells where SA2 is only phosphorylated on a single residue (see Figure 1). There are few if any cross reacting bands detected, indicating that the 1746 antibodies are mono-specific for SA2 when probed against a complex protein mixture as well as when probed against purified cohesin. These results are consistent with the notion that the reactivity of the antibodies with SA2 depends on mitotic SA2.

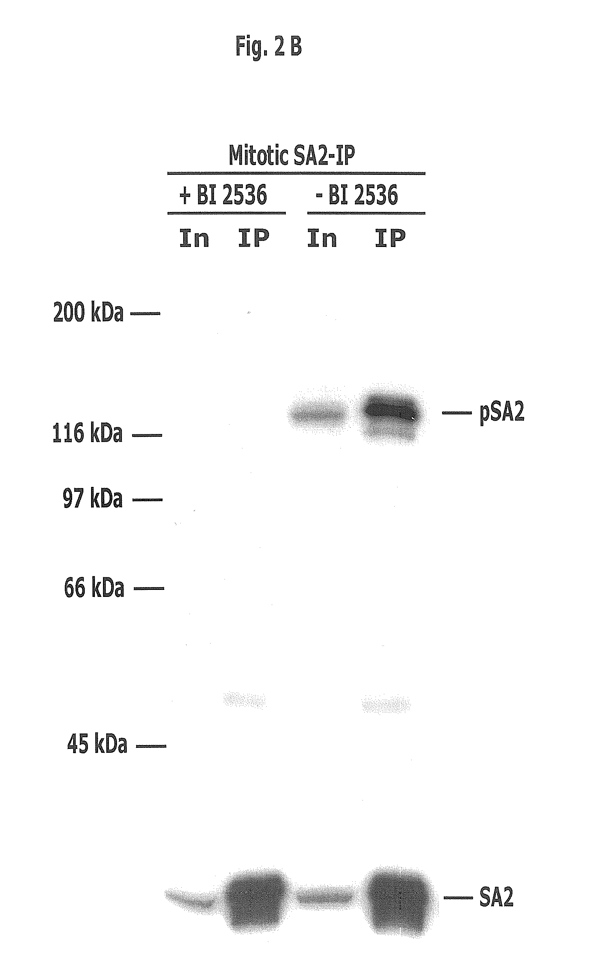

Second, to test whether the phosphorylation site that is detected specifically on mitotic SA2 is dependent on P1k1 in vivo, SA2 is immunopurified using the 446 antibodies from nocodazole-arrested cells that are treated or not with the P1k1 inhibitor BI 2536 as described in example 1, and the immunoprecipitates are analysed by SDS-PAGE and western blotting with the 1746 antibodies as described above. Figure 2B shows that 1746 antibodies do not recognise mitotic SA2 after treatment of mitotic cells with BI 2536. This indicates that the generation of the phosphorylation site detected by the 1746 antibodies is dependent on P1k1 in vivo.

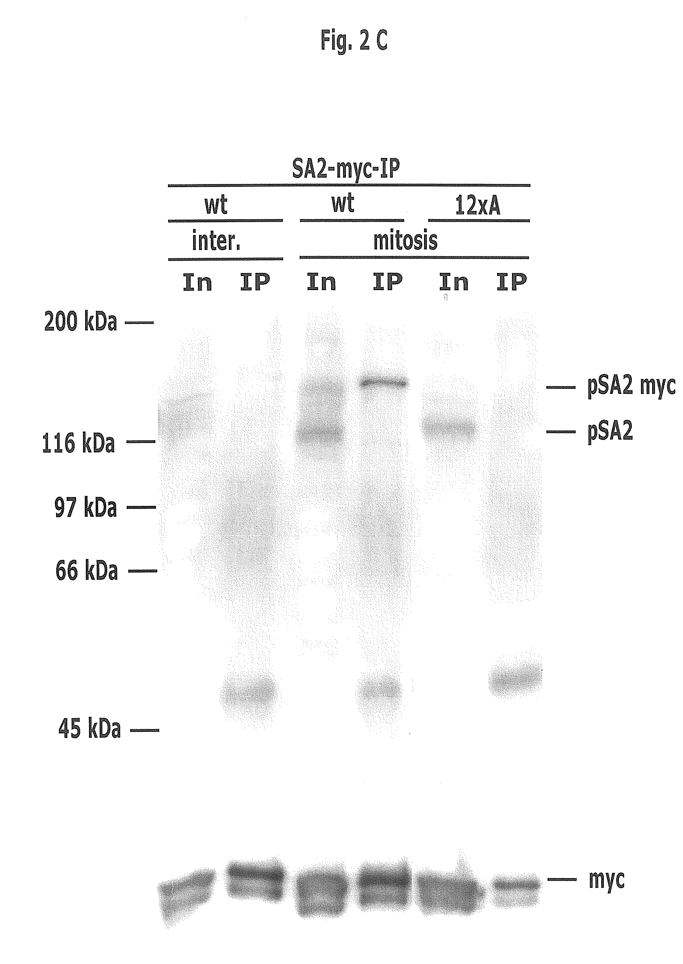

Third, to test whether the 1746 antibodies specifically recognises SA2 that is phosphorylated on one of the described residues (Hauf et al. 2005), a 9 myc tagged form of SA2 (SA2-myc) with either the wild type sequence or a mutated sequence in which 12 potential phosphorylation sites have been exchanged from serine /threonine to alanine (SA2-12xA-myc) is purified using a polyclonal myc antibodies (Gramsch laboratories) from interphase or nocodazole-arrested cells stably expressing the tagged SA2 (Hauf et al. 2005). The purified wild type and mutant SA2-myc as well as proteins present in the low speed supernatant are separated by SDS-PAGE and analysed by western blotting with 1746 antibodies as described above. As shown in figure 2C the 1746 antibodies specifically recognise endogenous SA2 and wild type SA2-myc in mitotic low speed supernatants and only the myc-tagged SA2 in anti-myc immunoprecipitates. In low speed supernatants from nocodazole-arrested cells expressing the 12xA mutant form of SA2-myc and in myc immunoprecipitates from these extracts, SA2-12xA-myc is not recognised by the 1746 antibodies while the endogenous SA2 is still detected in the low speed supernatant. These results clearly show that the 1746 antibodies react with SA2 only when it is phosphorylated on one of the previously described phosphorylation sites.

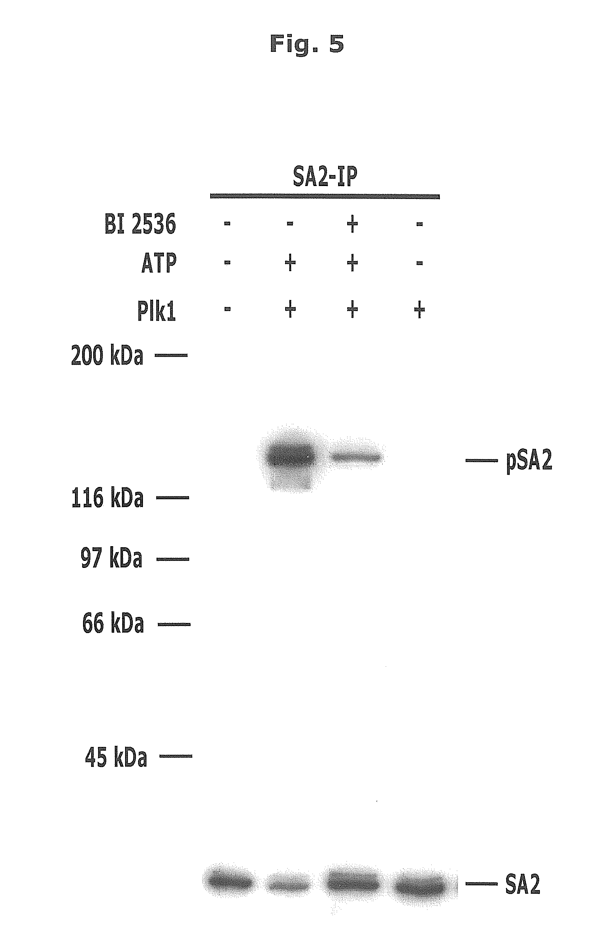

Fourth, to test whether the reactivity of the 1746 antibodies with SA2 depends directly on SA2 phosphorylation by P1k1 the following experiment is performed. Immunoprecipitated interphase SA2 is incubated in buffer containing MgCl₂ and recombinant active P1k1 enzyme supplemented or not with ATP and BI 2536. Subsequently, SA2 is analysed by SDS-PAGE and immunoblotting with the 1746 antibodies as described above. As is shown in Figure 5 the pre-treatment of interphase WAPL with MgCl₂, ATP and P1k1 allows the antibodies to react with SA2, whereas interphase SA2 pre-incubated with MgCl₂, P1k1 ATP and BI 2536 generates a much weaker signal. Also, interphase SA2 pre-incubated with MgCl₂ and P1k1 in the absence of ATP is not recognised by 1746. These data imply that the reactivity of the antibodies with SA2 directly depends on the prior phosphorylation of SA2 by P1k1 in vitro.

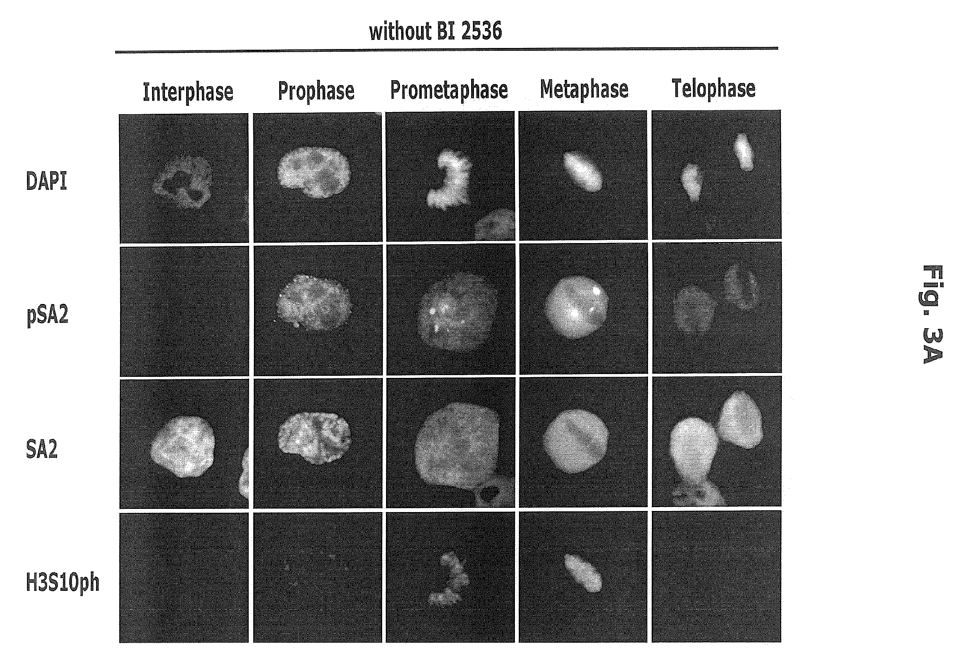

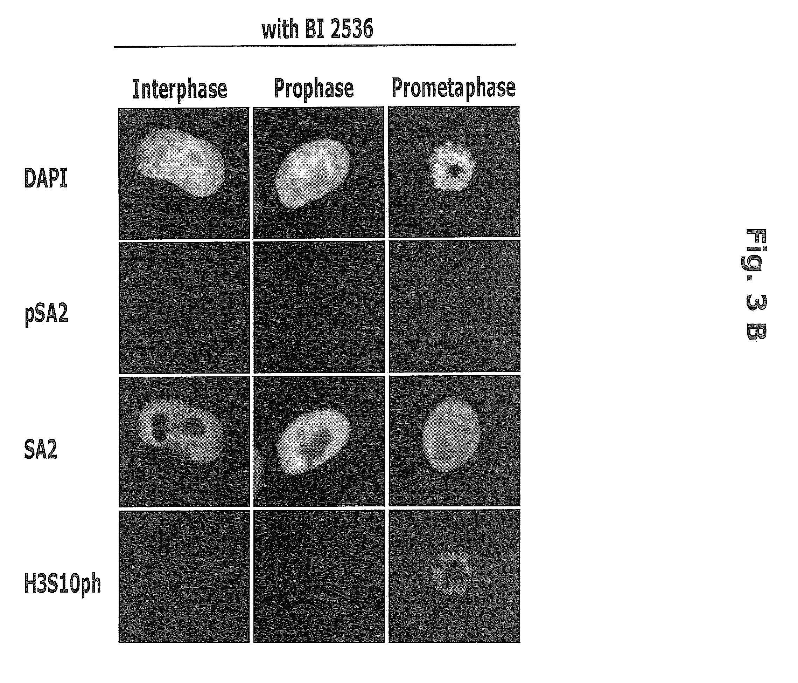

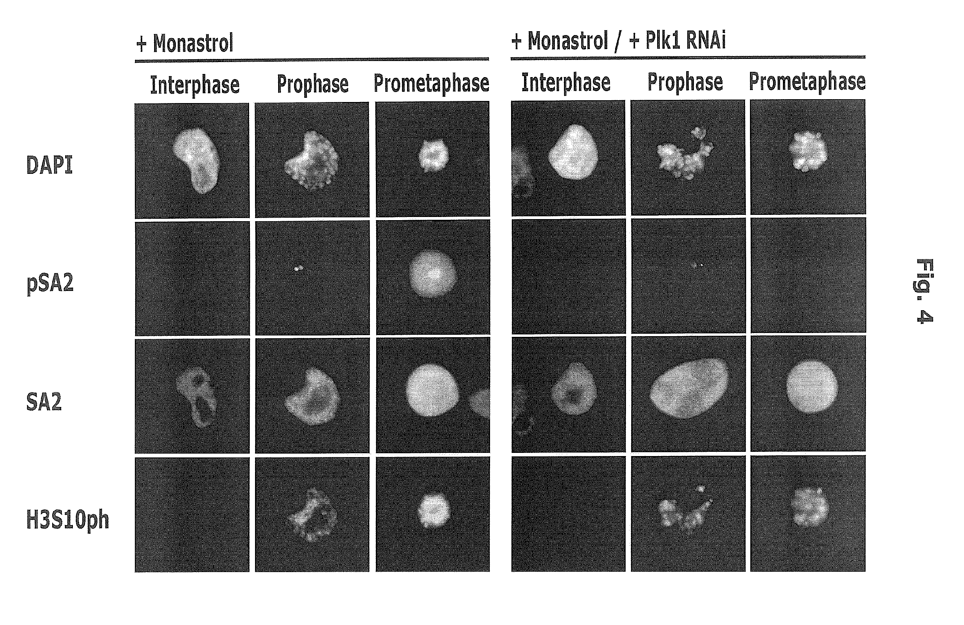

Fifth, to test whether the 1746 antibodies can also specifically recognise mitotic cells in immunocytological stainings, the following experiments are performed. HeLa cells are grown on cover slips, treated with BI 2536 for 45 minutes or not, fixed with paraformaldehyde and stained with the 1746 antibodies, antibodies that recognize SA2 independent of its phosphorylation state (anti-SA2, Bethyl), anti histone H3-phospho-Ser10 (H3S10ph) antibodies (Cell Signalling) as well as with DAPI and analysed by immunofluorescence microscopy. Representative images of stained cells in interphase and the mitotic phases prophase, prometaphase, metaphase and telophase are shown in figure 3A. The 1746 antibodies stain cells from prophase to metaphase but not cells in telophase nor cells in interphase. The 1746 localisation during mitosis resembles the SA2 localisation. It localises to the nucleus in prophase and can be detected throughout the cells after nuclear envelope breakdown in prometaphase until metaphase. The cells that have been treated with BI 2536 prior to fixation, staining and analysis do not show any staining with the 1746 antibodies in any of the cell cycle phases (figure 3B). Since cells treated with BI 2536 arrest at prometaphase, cells in metaphase and telophase are not present in this experiment. These data show that the 1746 antibodies recognise a BI 2536 dependent mitotic signal also in immunocytological stainings.
To confirm that the co-localisation of the 1746 antibodies with the SA2 antibodies really depends on SA2, SA2 is depleted from cells by siRNA. Cells are then fixed, stained with the 1746 antibodies, Scc1 antibodies and DAPI and analysed by immunofluorescence microscopy as described above. The 1746 signal decreased in response to SA2 depletion (data not shown), indicating that the 1746 antibodies recognise predominantly phosphorylated SA2 in fixed cells.
To confirm that the effect of BI 2536 on the 1746 signal in fixed mitotic cells really depends on P1k1, P1k1 is depleted by a siRNA oligonucleotide specific to P1k1 prior to fixation and staining with 1746 antibodies, DAPI, anti-SA2 antibodies (Bethyl) and H3S10ph antibodies (Cell Signalling). Stained cells are analysed as described above. Most cells depleted of P1k1 arrested at prometaphase (Sumara et al. 2004), metaphase and telophase cells are therefore not present after transfection with P1k1 siRNA. Just like in BI 2536- treated cells, the 1746 signal is reduced in P1k1 siRNA- treated cells from all cell cycle phases (figure 4). These results confirm that the generation of the phosphorylated form of SA2 that the 1746 antibodies recognise in fixed mitotic cells is dependent on P1k1.

### Figure 2:

(A) Low speed supernatant of HeLa cell extracts (In) and immunopurified SA2 (IP) from either exponentially growing cells or noc arrested cells are separated by SDS-PAGE, transferred to a PVDF membrane, probed with the pSA2 antibodies and, after stripping the membrane, with SA2 antibodies to control for equal loading.
(B) Low speed supernatant of HeLa cell extracts (In) and immunopurified SA2 (IP) from noc arrested cells treated or not with BI 2536 are separated by SDS-PAGE, transferred to a PVDF membrane, probed with pSA2 and, after stripping, with SA2 antibodies to control for equal loading.
(C) Low speed supernatant of HeLa cell extracts (In) and immunopurified SA2-myc (IP) from either exponentially cells or noc-arrested SA2-myc-wt or SA2-myc-12xA cell lines are separated by SDS-PAGE, transferred to a PVDF membrane, probed with pSA2 and, after stripping, with myc antibody to control for equal loading.

### Figure 3:

(A) HeLa Kyoto cells are grown on cover slips, fixed, stained with DAPI, pSA2, SA2 and H3S10ph and analysed by immunofluorescence microscopy. Representative cells from each cell cycle state are shown.
(B) HeLa Kyoto cells are grown on cover slips, treated with BI 2536 for 45 minutes, fixed, stained with DAPI, pSA2, SA2 and H3S10ph and analysed by immunofluorescence microscopy. Representative cells from each cell cycle state are shown.

### Figure 4:

HeLa Kyoto cells are grown on cover slips, treated or not with P1k1 siRNA oligonucleotides, treated with 0.1 mM monastrol for five hours, fixed, stained with DAPI, pSA2, SA2 and H3S10ph and analysed by immunofluorescence microscopy. Representative interphase, prophase and prometaphase arrest cells are shown.

### Figure 5:

SA2 is immunopurified by the 446 antibodies from extracts of exponentially growing HeLa cells, in vitro phosphorylated with recombinant P1k1 in presence or absence of either BI 2536 or ATP, separated by SDS-PAGE, transferred to a PVDF membrane, probed with pSA2 and, after stripping, with SA2 antibody to control for equal loading. S indicates immunopurified SA2 before the kinase assay.

### Example 3

A combination of chemical biology and mass spectrometry to detect P1k1 dependent phosphorylation sites in the cohesin complex.

Based on previous studies (Sumara et al. 2002, Hauf et al. 2005) it is suspected that the Scc1 and SA2 cohesin subunits are phosphorylated in mitosis in vivo by P1k1. To test this, cohesin is analysed in an experiment combining chemical biology and mass spectrometry as describe below.

HeLa cells are grown to 90% confluency and arrested in mitosis by nocodazole for a total of 16 hours. The P1k1 inhibitor BI 2536 is added 14 hours after nocodazole for a total of two hours. Control cells are left untreated. Mitotic cells are then harvested by mitotic shake off to separate them from interphase cells. To remove cell culture medium, cells are washed two times with ice cold PBS, frozen in liquid nitrogen and then stored at - 80°C. To extract soluble proteins from the cell pellets, they are resuspended in lysis buffer (buffer A) containing 20 mM Tris-HCl, pH 7.5; 150 mM NaCl, 5 mM EDTA, 20 mM beta-glycerophosphate, 10 mM NaF, 10 % glycerol, 0.1 % NP-40, 1 µM Okadaic acid, 0.2 mM NaVO4, 1 mM DTT and a protease inhibitor mix (Chymostatin, Leupeptin and Pepstatin at 10 µg / ml). Cells are lysed by extraction in a glass homogeniser for 10 minutes. Unsoluble proteins are removed by spinning twice at 13,000 rpm for 15 minutes. The supernatant is added to Affiprep beads (Amersham) containing crosslinked 446 antibodies and rotated overhead at 4°C for 1 hour followed by 6 extensive washes in buffer A and 5 washes in TBS to remove detergent. Proteins are eluted by adding 1.5x bead volumes of 0.2 M glycine pH 2.0. Eluates are adjusted to pH 8 by addition of 1.5 M Tris pH 9.2, subjected to reduction by 0.5 mg DTT / ml for 30 minutes at 56°C and alkylation by 2.5 mg / ml Iodoacetamide for 30 minutes. Samples are then digested in parallel with either 50 ng / ml of trypsin, chymotrypsin or GluC endoproteases for 16 hours, 4 hours or 16 hours respectively. Each digest is analysed by LC-ESI-MS/MS followed by spectra evaluation as described in materials and methods.

Detected phosphopeptides from the nocodazole - arrested sample and the nocodazole - arrested sample treated with BI 2536 are compared. Phosphorylated peptides which are present in both samples are considered to be P1k1 independent. Phosphorylated peptides which are not present in the BI 2536 - treated sample but are present in the control sample are considered P1k1 dependent only if the corresponding unphosphorylated peptide is detected in the BI 2536 - treated sample. In the analysed samples, the detected peptides cover 76% and 80% of the SA2 sequence, 74% and 73 % of the Scc1 sequence, 88% and 88% of Smc1 as well as 89% and 93% for Smc3 in the untreated or treated sample respectively. While there are no P1k1 dependent sites on Smc1 or Smc3, the analysis shows that the phosphorylation sites on serine 1160 or threonine 1161 as well as the phosphorylation site on serine 1224 or serine 1229 on SA2 are dependent on P1k1. In addition, the phosphorylation site on serine 134 (or serine 138) and the phosphorylation site on serine 545 on Scc1 are dependent on P1k1.

### References

Barr FA, Sillje HH, Nigg EA (2004) Polo-like kinases and the orchestration of cell division. Nat Rev Mol Cell Biol 5(6): 429-440.
Beck HC, Nielsen EC, Matthiesen R, Jensen LH, Sehested M, Finn P, Grauslund M, Hansen AM, Jensen ON (2006). Mol Cell Proteomics 1314-25.
Bulaj G, Kortemme, T. and Goldenberg, D. P. (1998). Ionization-reactivity relationships for cysteine thiols in polypeptides. Biochemistry 37, 8965-8972.
Cantin GT, Yates JR 3rd (2004). J Chromatogr A. 1053(1-2):7-14.
Ditchfield C., Johnson VL., Tighe A., Ellston R., Haworth C., Johnson T., Mortlock A., Keen N., Taylor SS. (2003). Aurora B couples chromosome alignment with anaphase by targeting BubR1, Mad2, and Cenp-E to kinetochores. J Cell Biol. Arp. 28, 161(2):267-80.
Druker and Lydon, J Clin Invest. (2000) Jan;105(1):3-7.
Ellman GL. (1959), Tissue sulfhydryl groups. Arch Biochem Biophys., May, 82(1):70-7.
Fry, D.W., Bedford, D.C., Harvey, PH., Fritsch, A., Keller, PR., Wu, Z., Dobrusin, E., Leopold, W.R., Fattaey (2001) J Biol Chem 276, 16617-16623.
Garin-Chesa, P., Old LJ., Rettig WJ., (1990), Cell surface glycoprotein of reactive stromal fibroblasts as a potential antibody target in human epithelial cancers. Proc Natl Acad Scie USA Sep 87(18):7235-9.
Giménez-Abian JF, Sumara I, Hirota T, Hauf S, Gerlich D et al. (2004). Regulation of Sister Chromatid Cohesion between Chromosome Arms. Curr Biol. 2004 Jul 13; 14(13):1187-93.
Graves, P.R., Yu, L., Schwarz, J.K., Gales, J., Sausville, E.A., O'Connor, P.M., Piwnica-Worms, H. (2000). J. Biol. Chem 275, 5600-5605.
Greiner D, Bonaldi T, Eskeland R, Roemer E, Imhof A (2005). Nat Chem Biol 143-5
Harlow, E. and Lane, D. (Eds.), (1988). Antibodies. A laboratory manual. Cold Spring Harbor laboratory Press. N.Y.
Hauf S, Cole RW, LaTerra S, Zimmer C, Schnapp G et al. (2003). The small molecule Hesperadin reveals a role for Aurora B in correcting kinetochore-microtubule attachment and in maintaining the spindle assembly checkpoint. J Cell Biol 161(2): 281-294.
Hauf S., Roitinger E., Koch B., Dittrich CM., Mechtler K., Peters JM. (2005). Dissociation of cohesin from chromosome arms and loss of arm cohesion during early mitosis depends on phosphorylation of SA2. PLoS Biol., Mar. 3(3):e69.
Hirota T, Gerlich D, Koch B, Ellenberg J, Peters JM (2004).J Cell Sci. 117(Pt 26):6435-45
Kennet, (1980)."Monoclonal Antibodies, Hybridomas--A New Dimension in Biological Analysis", Eds. Kennet, McKern and Bechtol, Plenum Press, N.Y. (1980).
Kitada et al., Mol. Cell. Biol. 1993, 13: 4445-4457;
Kohler and Milstein, (1975), Nature 256: 495.
Kraft C, Herzog F, Gieffers C, Mechtler K, Hagting A et al. (2003). Mitotic regulation of the human anaphase-promoting complex by phosphorylation. Embo J 22(24): 6598-6609.
Lane and Nigg, J. Cell Biol. 1996, 135: 1701-1713.
Lewis at al. (1994). THE JOURNAL OF BIOLOGICAL CHEMISTRY, Vol. 269, No. 42, Issue of October 21, pp. 26259-26266.
Llamazares et al., Genes Dev. 1991, 5: 2153-2165.
Liu and Erikson, PNAS 2002, 99: 8672-8676.
Liu and Erikson, PNAS 2003, 100: 5789-5794.
Losada A., Hirano M., Hirano T. (1998) Identification of Xenopus SMC protein complexes required for sister chromatid cohesion. Genes Dev. Jul 1., 12(13):1986-97.
Losada A, Hirano M, Hirano T (2002). Cohesin release is required for sister chromatid resolution, but not for condensin-mediated compaction, at the onset of mitosis. Genes Dev 16(23): 3004-3016.
Meijer L, Borgne A, Mulner O, Chong JP, Blow JJ, Inagaki N, Inagaki M, Delcros JG, Moulinoux JP (1997).Eur J Biochem. 243(1-2):527-36.
Minucci S, Horn V, Bhattacharyya N, Russanova V, Ogryzko VV, Gabriele L, Howard BH, Ozato K (1997). Proc Natl Acad Sci U S A 94(21):11295-300.
Rice JC, Briggs SD, Ueberheide B, Barber CM, Shabanowitz J, Hunt DF, Shinkai Y, Allis CD (2003).Mol Cell 12(6):1591-8.
Rena et al. (1999). J Biol Chem, June 11, Vol. 274, Issue 24, 17179-17183.
Spankuch-Schmitt et al., Oncogene 2002, 21: 3162-3171.
Strebhardt K., Ullrich A. (2006). Targeting polo-like kinase 1 for cancer therapy. Nat Rev Cancer, Apr. 6(4):321-30.
Sumara I, Vorlaufer E, Gieffers C, Peters BH, Peters JM (2000). Characterization of vertebrate cohesin complexes and their regulation in prophase. J Cell Biol 151(4): 749-762.
Sumara I, Vorlaufer E, Stukenberg PT, Kelm O, Redemann N et al. (2002). The dissociation of cohesin from chromosomes in prophase is regulated by Polo-like kinase. Mol Cell 9(3): 515-525.
Sumara et al., Curr. Biol. 2004, 14: 1712-1722).
van Vugt MA., van de Weerdt BC., Vader G., Janssen H., Calafat J., Klompmaker R., Wolthuis RM., Medema RH. (2004). Polo-like kinsase-1 is required for bipolar spindle formation but is dispensable for anaphase promoting complex/Cdc20 activation and initiation of cytokineses. J Biol Chem. Aug. 27; 279(35):36841-54.
Waizenegger IC, Hauf S, Meinke A, Peters JM (2000). Two distinct pathways remove mammalian cohesin from chromosome arms in prophase and from centromeres in anaphase. Cell 103(3): 399-410.
Wang, Y, Li, J., Booher, R.N., Kraker, A., Lawrence, T., Leopold, W.R. and Sun, Y (2001). Cancer Res 61, 8211-8217.
Wisdom, G.B., (1994), Peptide Antigens, A Practical Approach, Oxford University Press, Eds. Rickwood D. And Hames B.D.
Yates JR, 3rd, Link AJ, Schieltz D (2000). Direct analysis of proteins in mixtures. Application to protein complexes. Methods Mol Biol 146: 17-26.
Yuan, J., Horlin, A., Hock, B., Stutte, HJ., Rubsamen-Waigmann, H., and Strebhardt, K. (1997). American Journal of Pathology, Apr;150(4):1165-72.

## Claims

1. An antibody that binds to a phosphorylation site of the human cohesin subunit Scc1 (SEQ ID NO: 1) or of the human cohesin subunit SA2 (SEQ ID NO:2) when said phosphorylation site of Scc1 or SA2 is phosphorylated by P1k1 and that does not bind to said phosphorylation site when said phosphorylation site is not phosphorylated by P1k1.

2. The antibody of claim 1 that does not bind to said potential phosphorylation site when said phosphorylation by P1k1 is inhibited.

3. The antibody of claim 1 or 2 which is selected from a group of antibodies each of which binds to a phosphorylation site at
a. serine 134 (or serine 138, respectively) or serine 545 of Scc1 (SEQ ID NO: 1) or
b. threonine 1160 (or serine 1161, respectively) or serine 1224 (or serine 1229, respectively) on SA2 (SEQ ID NO:2).

4. A method for detecting P1k1 activity in a cell *ex vivo* or *in vitro* , wherein the presence of phosphorylated Scc1 and/or SA2 is determined by
a. contacting a biological sample suspected of P1k1 activity with one or more phospho-specific antibodies of any ones of claims 1 to 3 and
b. determining binding of said antibody or antibodies to Scc1 and/or SA2, wherein the amount of bound antibody is indicative of the level of P1k1 activity.

5. A method for determining a compound's effect on P1k1 activity in an individual treated with said compound by determining *ex vivo,* before and after administration of said compound to said individual, the presence of P1k1-dependent phosphorylation on Scc1 and/or SA2 in a sample, wherein
a. a sample obtained from said individual is contacted with one or more antibodies of any ones of claims 1 to 3,
b. binding of said antibody or antibodies to Scc1 and/or SA2 is determined, wherein the amount of bound antibody or antibodies is indicative of the level of P1k1 activity,
c. the levels of phosphorylated Scc1 and/or SA2 before and after administration of said compound are compared,
wherein a reduced level of phosphorylated Scc1 and/or SA2 upon administration of said compound is indicative of its inhibitory effect on P1k1.

6. The method of claim 5, wherein said compound's effect on P1k1 activity is determined over a period of time at pre-defined time points by comparing the levels of phosphorylated Scc1 and/or SA2 over said period of time, wherein a reduced level of phosphorylated Scc1 and/or SA2 is indicative of said compound's effect on P1k1 activity.

7. A method for selecting a cancer patient for being treated with a compound that acts as inhibitor of P1k1, by determining *ex vivo* the presence of phosphorylated Scc1 and/or SA2 in cells of said patient, wherein
a. a sample obtained from a tumor of said patient is contacted with one or more antibodies of any one of claims 1 to 3 and
b. binding of said antibody or antibodies to Scc1 and/or SA2 is determined,
wherein a high level of Scc1 and/or SA2 phosphorylation is indicative of aberrant P1k1 activity and a patient exhibiting such high phosphorylation level is selected for treatment with said P1k1 inhibitory compound.

8. The use of an antibody of any ones of claims 1 to 3 in a method for determining or confirming a compound's ability to inhibit P1k1 in a cell, wherein
a. a population of cells having P1k1 activity is adhered to a first solid support and contacted with a candidate compound for a period of time sufficient for the compound to exert its inhibitory effect on P1k1,
b. a capture antibody that binds to Scc1 or SA2 at a site different from a P1k1-dependent phosphorylation site is adhered to a second solid support,
c. a lysate of cells obtained from step a. is added to said second solid support to capture Scc1 or SA2, respectively,
d. unbound cell lysate is removed from the second solid support
e. the captured Scc1 or SA2 is exposed to a antibody of claims 1 or 2 that carries a detectable label,
f. binding of said labelled antibody to the captured Scc1 or SA2 is measured,
g. the signal obtained in step f. is compared with the signal obtained from control cells obtained without exposure of compound, wherein a reduction of the signal as compared to the control cells is indicative of said compound's inhibitory effect on P1k1.

9. A method for treating a patient or a patient population having a tumor that correlates with P1k1 activity, comprising the steps of
a) determining whether the tumor of said patient contains one or more P1k1-dependent phosphorylations,
b) selecting a patient or patient population whose tumor has one or more P1k1-dependent phosphorylation(s),
c) administering to said patient a therapeutically effective amount of a P1k1 inhibitor.

10. The method of claim 9, wherein step b) comprises determining one or more P1k1-dependent phosphorylations on the cohesin subunits Scc1 (SEQ ID NO: 1) and/or SA2 (SEQ ID NO:2),

11. The method of claim 9, wherein step b) comprises determining one or more P1k1-dependent phosphorylations
i) at serine 134 (or serine 138, respectively) or serine 545 of Scc1 (SEQ ID NO:1) or
ii) at threonine 1160 (or serine 1161, respectively) or serine 1224 (or serine 1229, respectively) on SA2 (SEQ ID NO:2).

12. The method of claim 9, wherein said P1k1 inhibitor administered in step c) is a compound of the formula


SEQUENCE LISTING

<110>  Boehringer Ingelheim International GmbH

<120>  Biomarkers for Polo-like kinase 1 activity

<130>  Case 14-0089

<160>  2

<170>  PatentIn version 3.1

<210>  1
<211>  631
<212>  PRT
<213>  Homo sapiens

<400>  1

<210>  2

<211>  1231

<212>  PRT

<213>  Homo sapiens

<400>  2

Met Ile Ala Ala Pro Glu Ile Pro Thr Asp Phe Asn Leu Leu Gln Glu
1               5                   10                  15

Ser Glu Thr His Phe Ser Ser Asp Thr Asp Phe Glu Asp Ile Glu Gly
            20                  25                  30

Lys Asn Gln Lys Gln Gly Lys Gly Lys Thr Cys Lys Lys Gly Lys Lys
        35                  40                  45

Gly Pro Ala Glu Lys Gly Lys Gly Gly Asn Gly Gly Gly Lys Pro Pro
    50                  55                  60

Ser Gly Pro Asn Arg Met Asn Gly His His Gln Gln Asn Gly Val Glu
65                  70                  75                  80

Asn Met Met Leu Phe Glu Val Val Lys Met Gly Lys Ser Ala Met Gln
                85                  90                  95

Ser Val Val Asp Asp Trp Ile Glu Ser Tyr Lys His Asp Arg Asp Ile
            100                 105                 110

Ala Leu Leu Asp Leu Ile Asn Phe Phe Ile Gln Cys Ser Gly Cys Lys
        115                 120                 125

Gly Val Val Thr Ala Glu Met Phe Arg His Met Gln Asn Ser Glu Ile
    130                 135                 140

Ile Arg Lys Met Thr Glu Glu Phe Asp Glu Asp Ser Gly Asp Tyr Pro
145                 150                 155                 160

Leu Thr Met Ala Gly Pro Gln Trp Lys Lys Phe Lys Ser Ser Phe Cys
                165                 170                 175

Glu Phe Ile Gly Val Leu Val Arg Gln Cys Gln Tyr Ser Ile Ile Tyr
            180                 185                 190

Asp Glu Tyr Met Met Asp Thr Val Ile Ser Leu Leu Thr Gly Leu Ser
        195                 200                 205

Asp Ser Gln Val Arg Ala Phe Arg His Thr Ser Thr Leu Ala Ala Met
    210                 215                 220

Lys Leu Met Thr Ala Leu Val Asn Val Ala Leu Asn Leu Ser Ile Asn
225                 230                 235                 240

Met Asp Asn Thr Gln Arg Gln Tyr Glu Ala Glu Arg Asn Lys Met Ile
                245                 250                 255

Gly Lys Arg Ala Asn Glu Arg Leu Glu Leu Leu Leu Gln Lys Arg Lys
            260                 265                 270

Glu Leu Gln Glu Asn Gln Asp Glu Ile Glu Asn Met Met Asn Ala Ile
        275                 280                 285

Phe Lys Gly Val Phe Val His Arg Tyr Arg Asp Ala Ile Ala Glu Ile
    290                 295                 300

Arg Ala Ile Cys Ile Glu Glu Ile Gly Ile Trp Met Lys Met Tyr Ser
305                 310                 315                 320

Asp Ala Phe Leu Asn Asp Ser Tyr Leu Lys Tyr Val Gly Trp Thr Met
                325                 330                 335

His Asp Lys Gln Gly Glu Val Arg Leu Lys Cys Leu Thr Ala Leu Gln
            340                 345                 350

Gly Leu Tyr Tyr Asn Lys Glu Leu Asn Ser Lys Leu Glu Leu Phe Thr
        355                 360                 365

Ser Arg Phe Lys Asp Arg Ile Val Ser Met Thr Leu Asp Lys Glu Tyr
    370                 375                 380

Asp Val Ala Val Gln Ala Ile Lys Leu Leu Thr Leu Val Leu Gln Ser
385                 390                 395                 400

Ser Glu Glu Val Leu Thr Ala Glu Asp Cys Glu Asn Val Tyr His Leu
                405                 410                 415

Val Tyr Ser Ala His Arg Pro Val Ala Val Ala Ala Gly Glu Phe Leu
            420                 425                 430

Tyr Lys Lys Leu Phe Ser Arg Arg Asp Pro Glu Glu Asp Gly Met Met
        435                 440                 445

Lys Arg Arg Gly Arg Gln Gly Pro Asn Ala Asn Leu Val Lys Thr Leu
    450                 455                 460

Val Phe Phe Phe Leu Glu Ser Glu Leu His Glu His Ala Ala Tyr Leu
465                 470                 475                 480

Val Asp Ser Met Trp Asp Cys Ala Thr Glu Leu Leu Lys Asp Trp Glu
                485                 490                 495

Cys Met Asn Ser Leu Leu Leu Glu Glu Pro Leu Ser Gly Glu Glu Ala
            500                 505                 510

Leu Thr Asp Arg Gln Glu Ser Ala Leu Ile Glu Ile Met Leu Cys Thr
        515                 520                 525

Ile Arg Gln Ala Ala Glu Cys His Pro Pro Val Gly Arg Gly Thr Gly
    530                 535                 540

Lys Arg Val Leu Thr Ala Lys Glu Lys Lys Thr Gln Leu Asp Asp Arg
545                 550                 555                 560

Thr Lys Ile Thr Glu Leu Phe Ala Val Ala Leu Pro Gln Leu Leu Ala
                565                 570                 575

Lys Tyr Ser Val Asp Ala Glu Lys Val Thr Asn Leu Leu Gln Leu Pro
            580                 585                 590

Gln Tyr Phe Asp Leu Glu Ile Tyr Thr Thr Gly Arg Leu Glu Lys His
            595                 600                 605

Leu Asp Ala Leu Leu Arg Gln Ile Arg Asn Ile Val Glu Lys His Thr
    610                 615                 620

Asp Thr Asp Val Leu Glu Ala Cys Ser Lys Thr Tyr His Ala Leu Cys
625                 630                 635                 640

Asn Glu Glu Phe Thr Ile Phe Asn Arg Val Asp Ile Ser Arg Ser Gln
                645                 650                 655

Leu Ile Asp Glu Leu Ala Asp Lys Phe Asn Arg Leu Leu Glu Asp Phe
            660                 665                 670

Leu Gln Glu Gly Glu Glu Pro Asp Glu Asp Asp Ala Tyr Gln Val Leu
        675                 680                 685

Ser Thr Leu Lys Arg Ile Thr Ala Phe His Asn Ala His Asp Leu Ser
    690                 695                 700

Lys Trp Asp Leu Phe Ala Cys Asn Tyr Lys Leu Leu Lys Thr Gly Ile
705                 710                 715                 720

Glu Asn Gly Asp Met Pro Glu Gln Ile Val Ile His Ala Leu Gln Cys
                725                 730                 735

Thr His Tyr Val Ile Leu Trp Gln Leu Ala Lys Ile Thr Glu Ser Ser
            740                 745                 750

Ser Thr Lys Glu Asp Leu Leu Arg Leu Lys Lys Gln Met Arg Val Phe
        755                 760                 765

Cys Gln Ile Cys Gln His Tyr Leu Thr Asn Val Asn Thr Thr Val Lys
    770                 775                 780

Glu Gln Ala Phe Thr Ile Leu Cys Asp Ile Leu Met Ile Phe Ser His
785                 790                 795                 800

Gln Ile Met Ser Gly Gly Arg Asp Met Leu Glu Pro Leu Val Tyr Thr
                805                 810                 815

Pro Asp Ser Ser Leu Gln Ser Glu Leu Leu Ser Phe Ile Leu Asp His
            820                 825                 830

Val Phe Ile Glu Gln Asp Asp Asp Asn Asn Ser Ala Asp Gly Gln Gln
        835                 840                 845

Glu Asp Glu Ala Ser Lys Ile Glu Ala Leu His Lys Arg Arg Asn Leu
    850                 855                 860

Leu Ala Ala Phe Cys Lys Leu Ile Val Tyr Thr Val Val Glu Met Asn
865                 870                 875                 880

Thr Ala Ala Asp Ile Phe Lys Gln Tyr Met Lys Tyr Tyr Asn Asp Tyr
                885                 890                 895

Gly Asp Ile Ile Lys Glu Thr Met Ser Lys Thr Arg Gln Ile Asp Lys
            900                 905                 910

Ile Gln Cys Ala Lys Thr Leu Ile Leu Ser Leu Gln Gln Leu Phe Asn
        915                 920                 925

Glu Met Ile Gln Glu Asn Gly Tyr Asn Phe Asp Arg Ser Ser Ser Thr
    930                 935                 940

Phe Ser Gly Ile Lys Glu Leu Ala Arg Arg Phe Ala Leu Thr Phe Gly
945                 950                 955                 960

Leu Asp Gln Leu Lys Thr Arg Glu Ala Ile Ala Met Leu His Lys Asp
                965                 970                 975

Gly Ile Glu Phe Ala Phe Lys Glu Pro Asn Pro Gln Gly Glu Ser His
            980                 985                 990

Pro Pro Leu Asn Leu Ala Phe Leu Asp Ile Leu Ser Glu Phe Ser Ser
        995                 1000                1005

Lys Leu Leu Arg Gln Asp Lys Arg Thr Val Tyr Val Tyr Leu Glu
    1010                1015                1020

Lys Phe Met Thr Phe Gln Met Ser Leu Arg Arg Glu Asp Val Trp
    1025                1030                1035

Leu Pro Leu Met Ser Tyr Arg Asn Ser Leu Leu Ala Gly Gly Asp
    1040                1045                1050

Asp Asp Thr Met Ser Val Ile Ser Gly Ile Ser Ser Arg Gly Ser
    1055                1060                1065

Thr Val Arg Ser Lys Lys Ser Lys Pro Ser Thr Gly Lys Arg Lys
    1070                1075                1080

Val Val Glu Gly Met Gln Leu Ser Leu Thr Glu Glu Ser Ser Ser
    1085                1090                1095

Ser Asp Ser Met Trp Leu Ser Arg Glu Gln Thr Leu His Thr Pro
    1100                1105                1110

Val Met Met Gln Thr Pro Gln Leu Thr Ser Thr Ile Met Arg Glu
    1115                1120                1125

Pro Lys Arg Leu Arg Pro Glu Asp Ser Phe Met Ser Val Tyr Pro
    1130                1135                1140

Met Gln Thr Glu His His Gln Thr Pro Leu Asp Tyr Asn Arg Arg
    1145                1150                1155

Gly Thr Ser Leu Met Glu Asp Asp Glu Glu Pro Ile Val Glu Asp
    1160                1165                1170

Val Met Met Ser Ser Glu Gly Arg Ile Glu Asp Leu Asn Glu Gly

    1175                1180                1185

Met Asp Phe Asp Thr Met Asp Ile Asp Leu Pro Pro Ser Lys Asn
    1190                1195                1200

Arg Arg Glu Arg Thr Glu Leu Lys Pro Asp Phe Phe Asp Pro Ala
    1205                1210                1215

Ser Ile Met Asp Glu Ser Val Leu Gly Val Ser Met Phe
    1220                1225                1230